# EUROPEAN PATENT SPECIFICATION

(11) **EP 2 129 401 B1**
(45) Date of publication and mention of the grant of the patent: **17.07.2019**
(21) Application number: 07863175.1
(22) Date of filing: 19.12.2007
(51) Int. Cl.: A61K 47/22, A61K 39/395, A61K 47/18, A61K 9/08

(54) **STABLE BUFFERED FORMULATIONS CONTAINING POLYPEPTIDES**
STABILE GEPUFFERTE FORMULIERUNGEN, DIE POLYPEPTIDE ENTHALTEN
FORMULATIONS TAMPONNÉES STABLES CONTENANT DES POLYPEPTIDES

(30) Priority: 21.12.2006 US 876801 P
(43) Date of publication of application: 09.12.2009
(73) Proprietor: Amgen, Inc, Thousand Oaks, California 91320-1799 (US)
(72) Inventor: JACOB, Jaby, Seattle, Washington 98107 (US); MATSUMURA, Masazumi, Thousand Oaks, California 91320-1799 (US); GOKARN, Yatin, Foster City, CA 94404 (US); BREMS, David, Newburry Park, California 91320 (US)
(74) Representative: Grünecker Patent- und Rechtsanwälte PartG mbB
(86) International application number: PCT/US2007/026060
(87) International publication number: WO 2008/079290

(56) References cited:
- EP-A- 1 712 240
- WO-A-96/07429
- WO-A-98/14476
- WO-A-2005/049078
- US-B1- 6 171 586
- Z. IGNATOVA ET AL: "Inhibition of protein aggregation in vitro and in vivo by a natural osmoprotectant" PROCEEDINGS OF TEH NATIONAL ACADEMY OF SIENCES, vol. 103, no. 36, 5 September 2006 (2006-09-05), pages 13357-13361, XP002495204 cited in the application
- ARAKAWA T ET AL: "PROTEIN-SOLVENT INTERACTIONS IN PHARMACEUTICAL FORMULATIONS" PHARMACEUTICAL RESEARCH, NEW YORK, NY, US, vol. 8, no. 3, 1 January 1991 (1991-01-01), pages 285-291, XP009052919 ISSN: 0724-8741

## Description

### FIELD OF THE INVENTION

This application relates generally to medicines for the treatment of diseases, for example, to formulations for biological molecule pharmaceuticals or biopharmaceuticals.

### BACKGROUND

With the advent of recombinant DNA technology and other advances in antibody production, protein-based therapeutics have become increasingly commonplace in the repertoire of drugs available to medical practitioners for the treatment of a wide range of diseases from cancer to autoimmune diseases. The ability to employ biological molecules, for example, antibodies or recombinant proteins, as pharmaceuticals in the treatment of diseases has advanced medical care and quality of life over the past quarter of a century. As of the year 2005, there were more than one hundred and fifty approved protein-based pharmaceuticals on the market and this number is expected to rise dramatically in the coming years.

Proteins known to exhibit various pharmacological actions *in vivo* are now capable of being produced in large amounts for various pharmaceutical applications. Long-term stability of a therapeutic protein, for example an antibody, is a particularly beneficial criterion for safe, consistent and efficacious treatments. Loss of functionality of the therapeutic within a preparation can decrease its effective concentration for a given administration. Similarly, undesired modifications of a therapeutic can affect the activity and/or the safety of a preparation.

Proteins are complex molecules with primary, secondary, tertiary and in some cases quaternary structures, all of which can play a role in imparting biological function. Structural complexity of biological pharmaceuticals such as proteins make them susceptible to various processes that can result in structural and functional instability as well as loss of safety. With respect to these instability processes or degradation pathways, a protein can undergo a variety of covalent and non-covalent reactions or modifications in solution. For example, protein degradation pathways can be generally classified into two main categories: (i) physical degradation pathways, and (ii) chemical degradation pathways.

Protein drugs can be susceptible to the physical degradation process of irreversible aggregation. Protein aggregation is of particular interest in biopharmaceutical production because it often results in diminished bioactivity that affects drug potency, and also can elicit immunological or antigenic reactions in patients. Chemical degradation of a protein therapeutic, including degradation of the chemical structure by, for example, chemical modification, also has been implicated in increasing a biopharmaceutical's immunogenic potential. Thus, stable protein formulations should minimize both physical and chemical degradation pathways of the drug of interest.

Proteins can degrade, for example, via physical processes such as interfacial adsorption and aggregation. Adsorption can impact a protein drug's potency and stability. It can cause a loss in potency of low concentration dosage forms. A second consequence is that unfolding mediated adsorption at interfaces can often be an initiating step for irreversible aggregation in solution. In this respect, proteins tend to adsorb at liquid-solid, liquid-air, and liquid-liquid interfaces. Sufficient exposure of a protein's core at a hydrophobic surface can result in adsorption as a consequence of agitation, temperature or pH induced stresses. Further, proteins can also be sensitive to, for example, pH, ionic strength, thermal, shear and interfacial stresses, all of which can lead to aggregation and result in instability.

Proteins can also be subject to a variety of chemical modification and/or degradation reactions, for example, deamidation, isomerization, hydrolysis, disulfide scrambling, beta-elimination, oxidation and adduct formation. The principal hydrolytic mechanisms of degradation can include peptide bond hydrolysis, deamidation of asparagine and glutamine and the isomerization of aspartic acid. Other degradation pathways can include beta-elimination reactions, which can occur under alkaline pH conditions and lead to racemization or loss of part of the side-chain for certain amino acids. Oxidations of methionine, cysteine, histidine, tyrosine and tryptophan residues can also occur.

Because of the number and diversity of different reactions that can result in protein instability, the composition of components in a formulation can affect the extent of protein degradation and, consequently, the safety and efficacy of the therapeutic. The formulation of a biopharmaceutical can also affect the ease and frequency of administration and amount of pain experienced by a patient upon injection. For example, immunogenic reactions have not only been attributed to protein aggregates but also to mixed aggregates of the therapeutic protein with an inactive component contained in the formulation Schellekens, H., Nat. Rev. Drug Discov. 1:457-62(2002); Hesmeling, et al., Pharm. Res. 22:1997-2006 (2005).

A formulation that retains longer-term stability relative to other formulations, under a variety of conditions could provide an effective means of delivering an efficacious and safe amount of the biopharmaceutical. Retention of longer-term stability in a formulation could also lower production and treatment costs. Numerous recombinant or natural proteins could benefit from such consistently stable formulations and thereby provide more effective clinical results.

Various formulations to stabilize biologically active proteins have appeared in the art. For example, Patent Publication No. US 2006/0024346 discusses aqueous solutions comprising a biologically active protein, a polysaccharide and an amino acid based compound and US Patent No. 6,171,586B discusses formulations comprising an antibody, an acetate buffer, a surfactant and a polyol, but lacking a tonicifying amount of sodium chloride. Patent Publication No. US 2005/0142139 discusses pharmaceutical formulations comprising a CD4-IgG2 chimeric heterotetramer, a histidine buffer, a non-ionic detergent and an amino acid that can comprise alanine, glycine, proline or glycylglycine. International Publication No. WO 2005/063291 A1 discusses a formulation comprising antibodies in a glutamate buffer. Additionally, International Publication No. WO 2005/44854 discusses formulations of acetic acid, glutamic acid or aspartic acid buffers containing an anti-CD40 antibody.

In another Patent Publication (No. 2003/0138417), pharmaceutical formulations comprising 50 mg/ml or more of antibody in either succinate or histidine buffer was proposed. Results of studies with either buffer, however, indicated that the amino acids, glycine, lysine, serine, proline or methionine did not have a stabilizing effect on protein in the formulation.

This application provides new formulations that retain increased stability of a biopharmaceutical under a variety of different manufacturing and storage conditions. Biopharmaceuticals used with formulations described in the specification can comprise, *inter alia,* therapeutic antibody formulations.

### SUMMARY

The present invention provides a formulation comprising an acetic acid buffer having a pH from about 4.0 to about 6.0, proline at a concentration of about 2% to about 10% (w/v), and an antibody or antigen-binding fragment, wherein the formulation does not further comprise both a polyol and a surfactant.

In another embodiment, the present invention provides a method of preparing a formulation comprising combining an acetic acid buffer having a pH from about 4.0 to about 6.0, proline and an effective amount of an antibody or antigen-binding fragment, wherein the formulation does not further comprise both a polyol and a surfactant.

In another embodiment, the present invention provides a container containing a formulation comprising an aqueous solution having between about 3 to about 50 mM acetic acid with a pH from about 4.0 to about 6.0, proline from about 2% to about 10% (w/v) and an antibody or antigen-binding fragment, wherein the aqueous solution does not further comprise both a polyol and a surfactant.

In another embodiment, the present invention provides a formulation for use in treating a condition caused by increased expression of nerve growth factor or increased sensitivity to nerve growth factor in a patient, comprising an acetic acid buffer having a pH from about 4.0 to about 6.0, proline at a concentration of about 2% to about 10% (w/v), and an effective amount of an antibody or antigen-binding fragment to nerve-growth factor, wherein the formulation does not further comprise both a polyol and a surfactant.

Preferred embodiments are set forth in the subclaims.

A formulation comprising a buffering solution, proline, and an effective amount of a biopharmaceutical according to claim 1 is provided. The specification also discloses a method of preparing the formulation, methods of treating a condition using the formulation, and a kit containing components of the formulation.

In various embodiments, the formulation comprises proline having a concentration of about 3% and a polypeptide having a concentration of about 3 mg/ml to about 50 mg/ml or about 100 mg/ml. The polypeptide is an antibody or antigen-binding fragment. The antibody or antigen-binding fragment can bind to growth factor, for example, nerve growth factor (NGF).

In various embodiments, the concentration of the buffering solution can be from about 1 mM to about 100 mM, from about 2 mM to about 50 mM, from about 3mM to about 30 mM, from about 4 mM to about 20 mM, or from about 5 mM to about 10 mM, from about 10 mM to about 40 mM, from about 15 mM to about 35 mM, from about 20 mM to about 30 mM, from about 25 mM to about 35 mM about, about 26 mM, about 27 mM, about 28 mM, about 29 mM, about 30 mM, about 31 mM, about 32 mM, about 33 mM or about 34 mM. The therapeutic polypeptide included in the formulation can comprise, an Fd, Fv, Fab, F(ab'), F(ab)₂, F(ab')₂, F(ab)₃, Fc, bis-scFv(s), single chain Fv (scFv), monoclonal antibodies, polyclonal antibodies, recombinant antibodies, chimeric antibodies, diabodies, triabodies, tetrabodies, minibody, peptibodies, VhH domain, V-NAR domain, V_{H} domain, V_{L} domain, camel Ig, Ig NAR, or receptibody or combinations of the above.

The present specification disclosese a method comprising combining an aqueous acetic acid buffer with a pH from about 4.0 to about 6.0 with proline and a therapeutic antibody, but not containing both a polyol and a surfactant.

In various embodiments, a formulation for use in a method is provided of treating a condition caused by increased expression of a growth factor or increased sensitivity to a growth factor in a patient, the method comprising administering to a patient a pharmaceutically effective amount of a formulation comprising an acetic acid buffer having a pH from about 4.0 to about 6.0, proline at a concentration of about 2% to about 10%, and an effective amount of an antibody to a growth factor or a growth factor-binding antigen-binding fragment according to claim 10. The condition being treated can be pain or neuropathic pain.

In various embodiments the condition caused by increased expression of a growth factor or increased sensitivity to a growth factor can result from increased expression of nerve growth factor. Therefore, the formulations described herein may comprise an antibody or antigen-binding fragment to nerve growth factor. The formulation can provide a treatment to pain or neuropathic pain.

The present specification further discloses a kit comprising a buffer, proline and an antibody. If the buffer is an acetic acid buffer, the kit does not contain both a polyol and a surfactant.

### BRIEF DESCRIPTION OF THE DRAWINGS

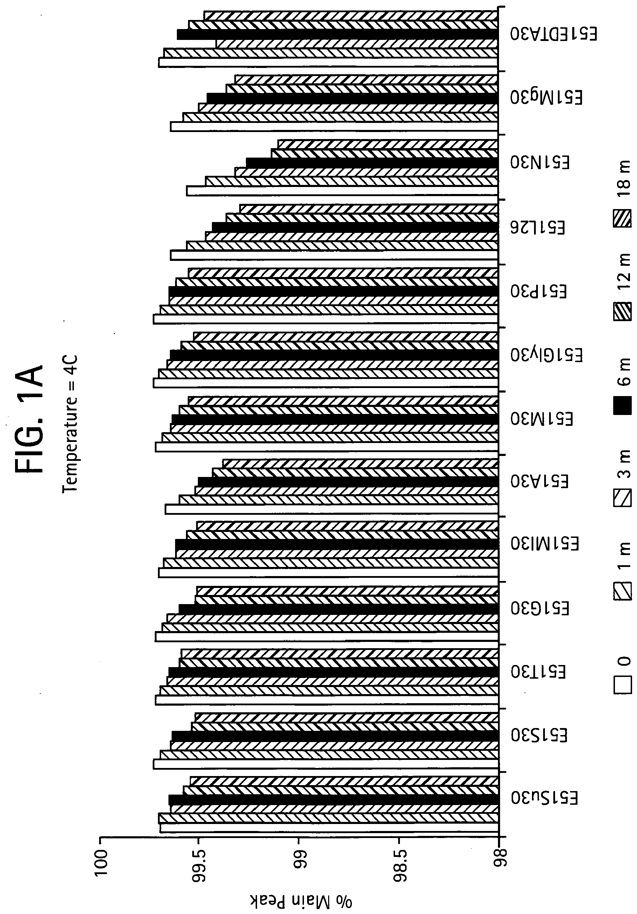

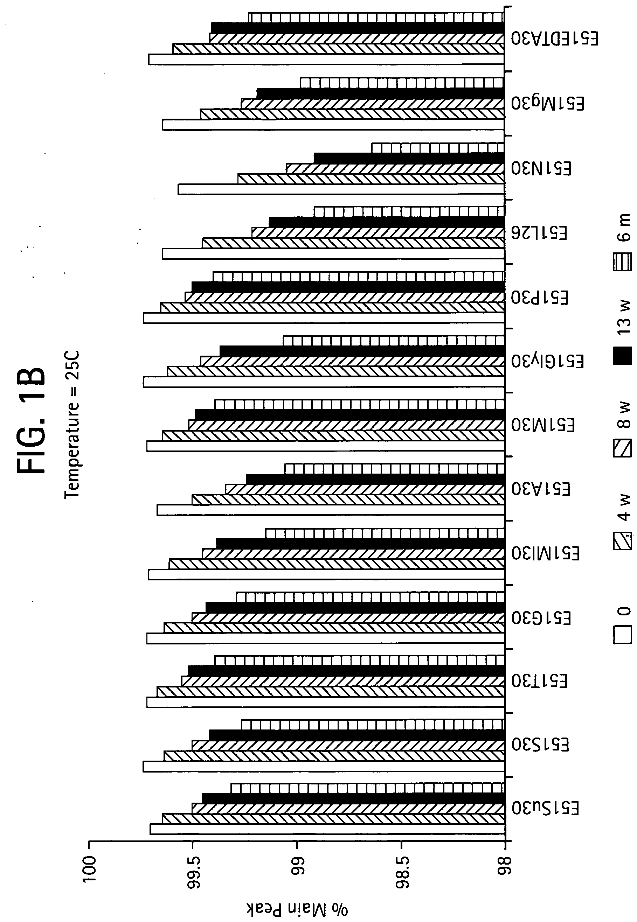

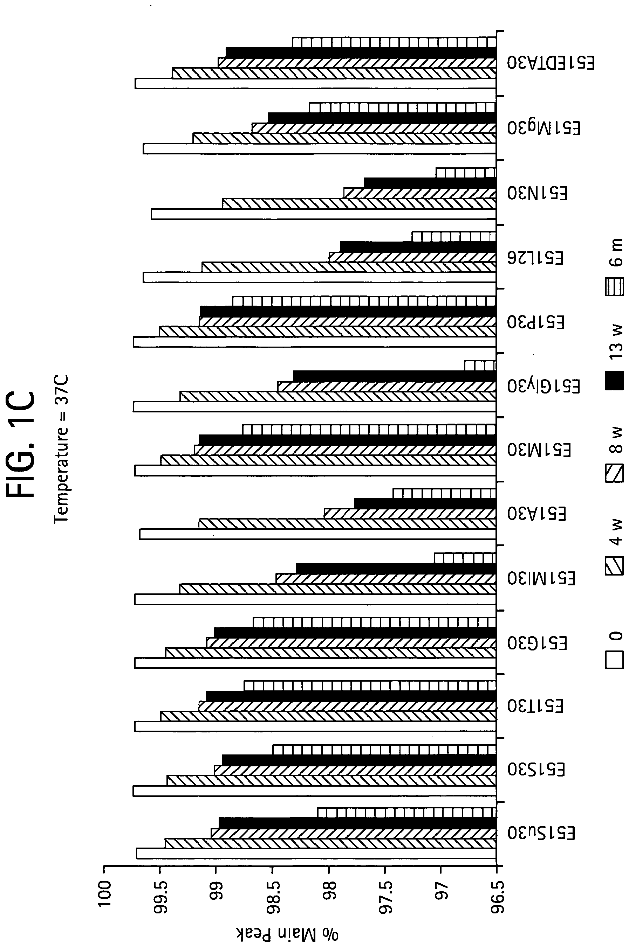

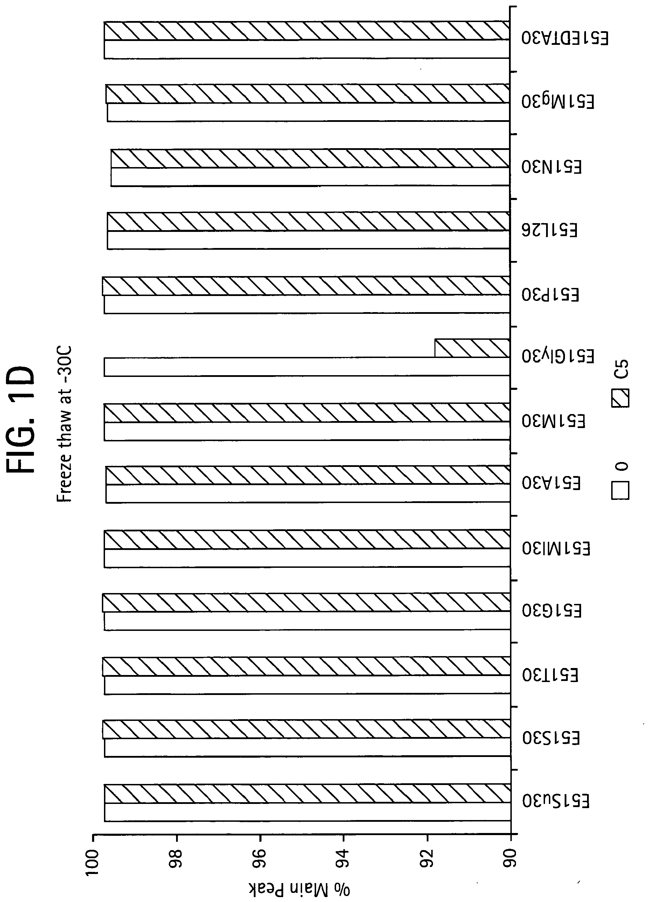

Experiments is Figures 1-7 use an IgG₂ antibody, while experiments in Figure 8 and 9 use an IgG₁ antibody
Figure 1A-1D illustrates results of size exclusion chromatography (SEC) experiment for different formulations under varying storage conditions for up to 18 months. Preparation E51P30 contains 10 mM L-glutamic acid buffer (pH 5.1), 3.1% L-proline and 30 mg/ml of antibody. Figure 1A illustrates results following storage at 4°C. Figure 1B illustrates results following storage at 25°C. Figure 1C illustrates results following storage at 37°C. Figure 1D illustrates results following freeze-thaw at -30°C. The results demonstrate that at most time points over several different temperatures, the E51P30 formulation usually displays the least loss in percentage of the main peak.

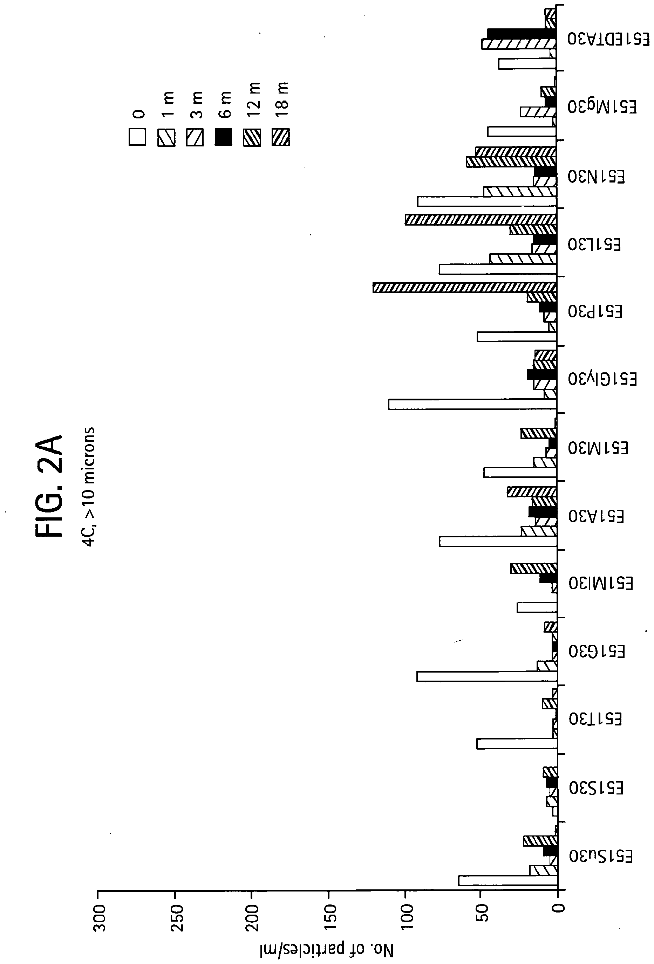

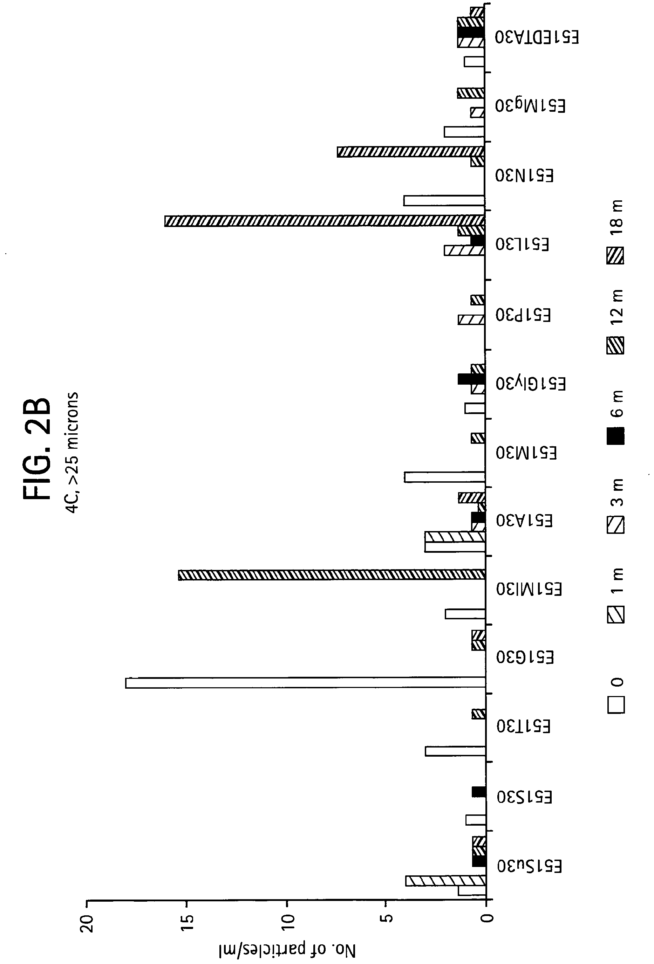

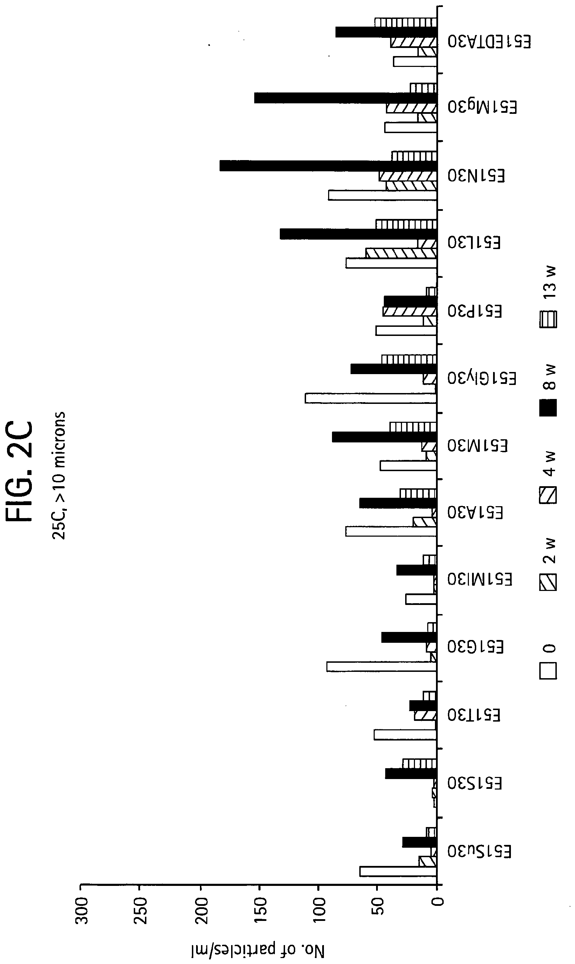

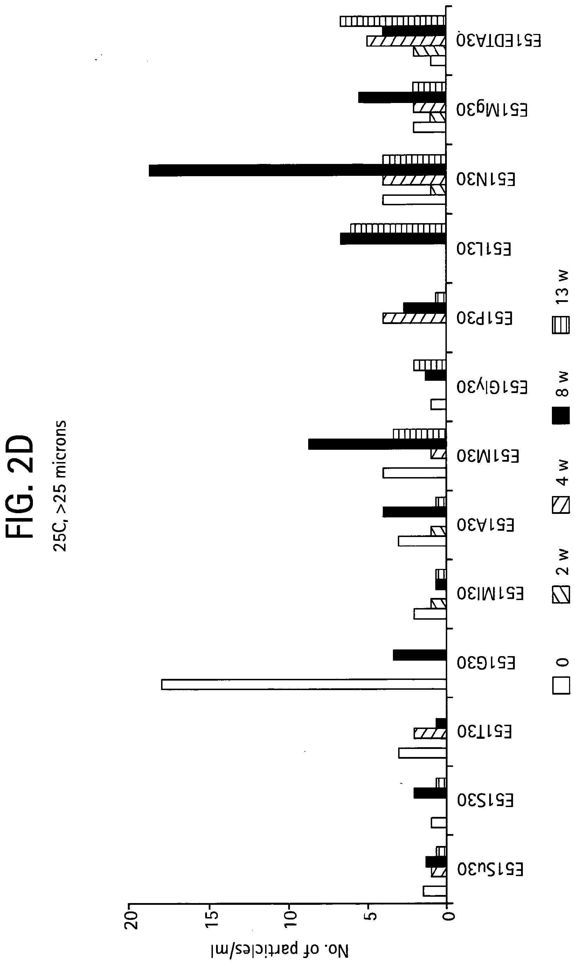

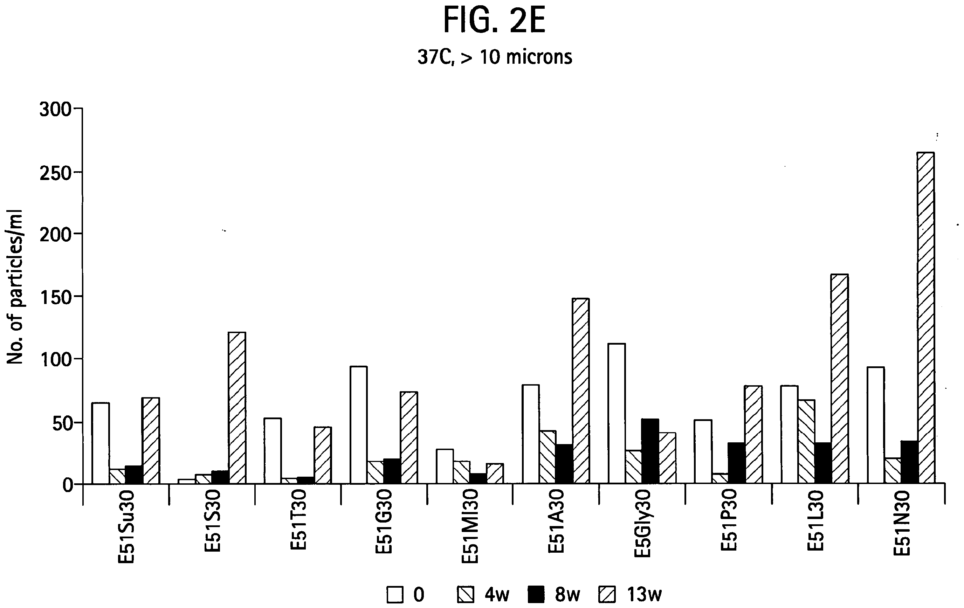

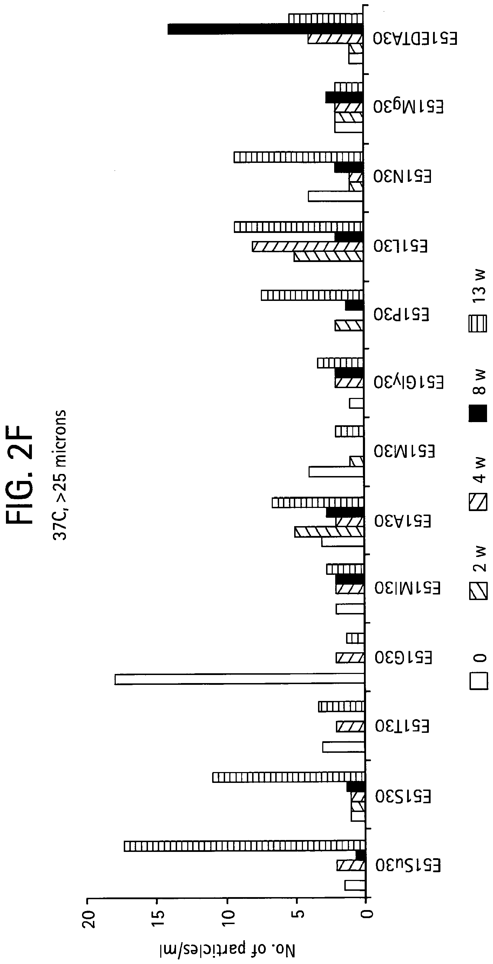

Figures 2A-2F illustrates results from measurement of the number of particles greater than 10 µm (Figures 2A, 2C and 2E) or 25 µm (Figures 2B, 2D and 2F) after storage at 4°C, 25°C or 37°C in various formulations. The formulations were analyzed by a HIAC Royco, liquid particle counting system, Model 9703 (Hach-Ultra, Grants Pass, OR, USA).

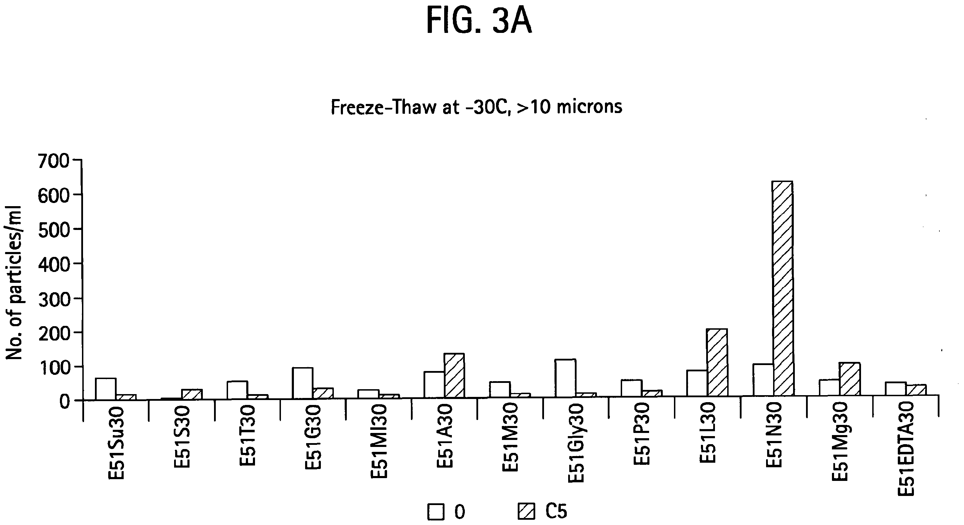

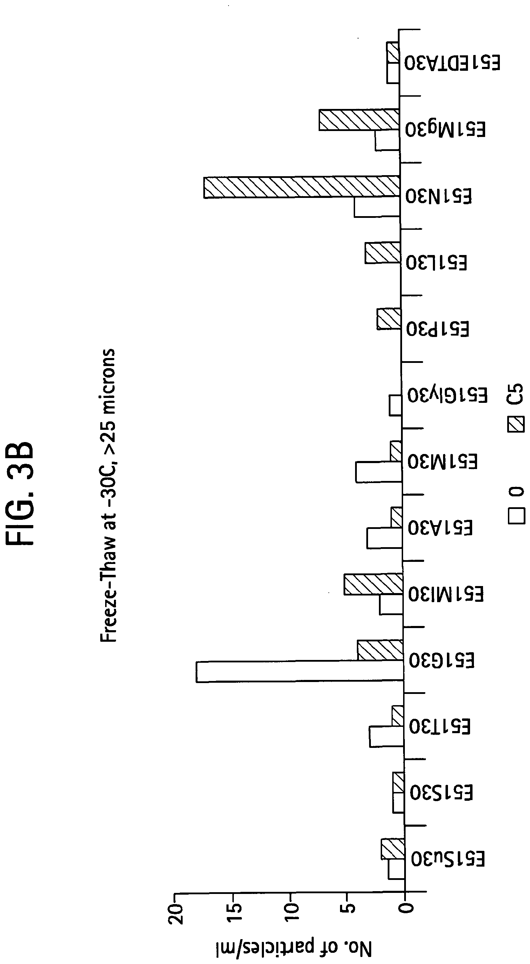

Figures 3A-3B illustrates the measurement of the number of particles formed after 5 cycles of freezing at -30°C and thawing at room temperature.

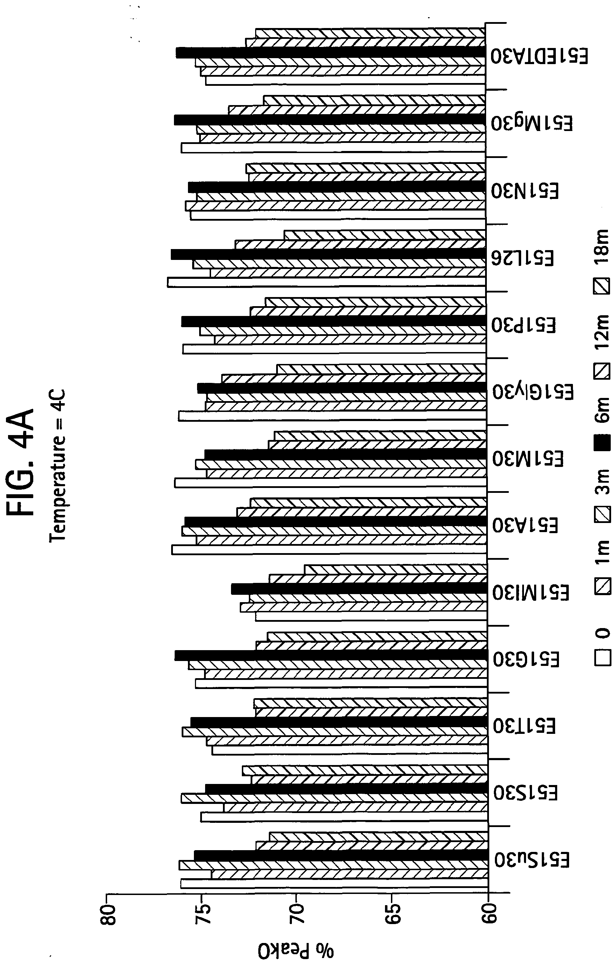

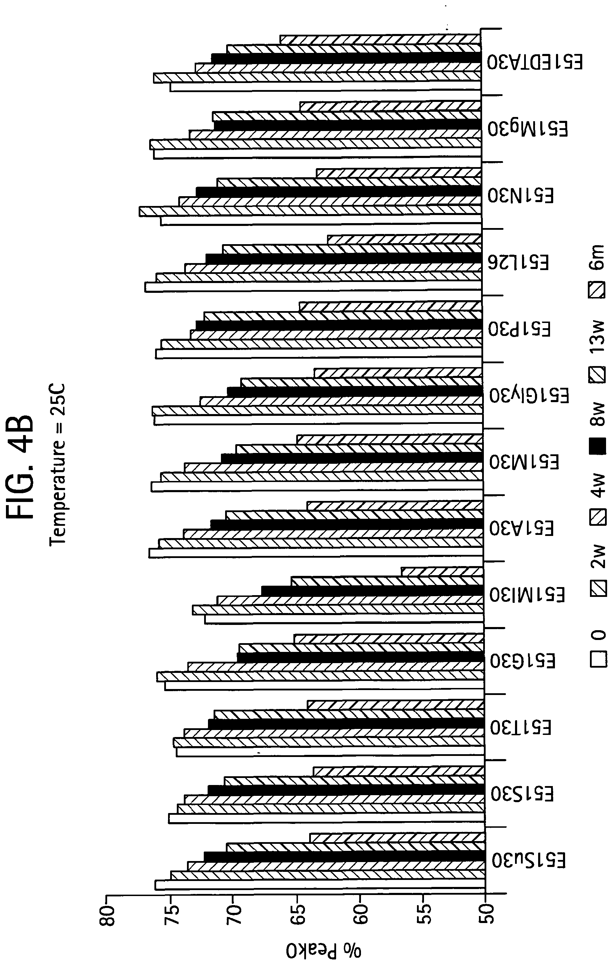

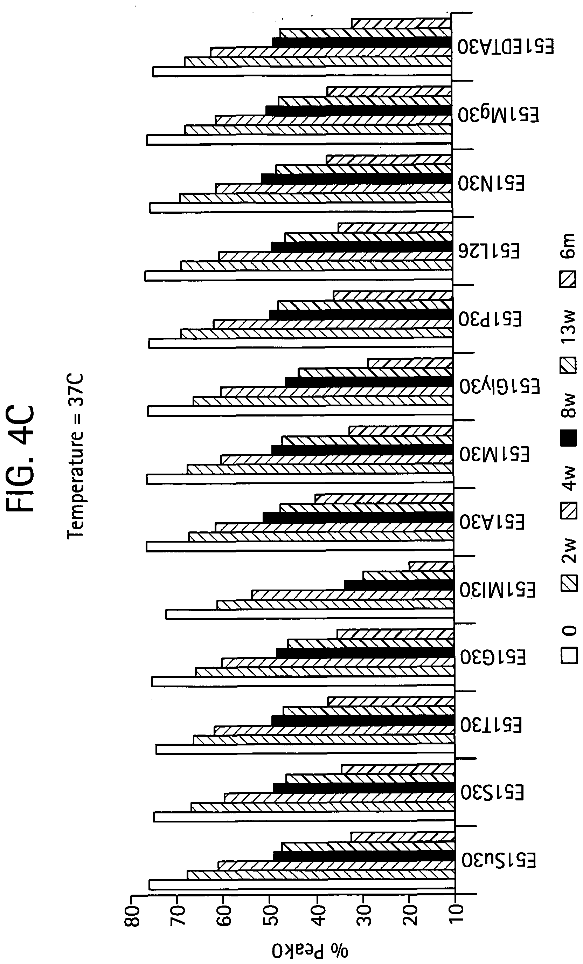

Figures 4A-4C illustrate changes in the percentage of the main peak (Peak-0) observed by weak cation exchange chromatography (CEX) after storage at 4°C (Figure 4A), 25°C (Figure 4B) and 37°C (Figure 4C).

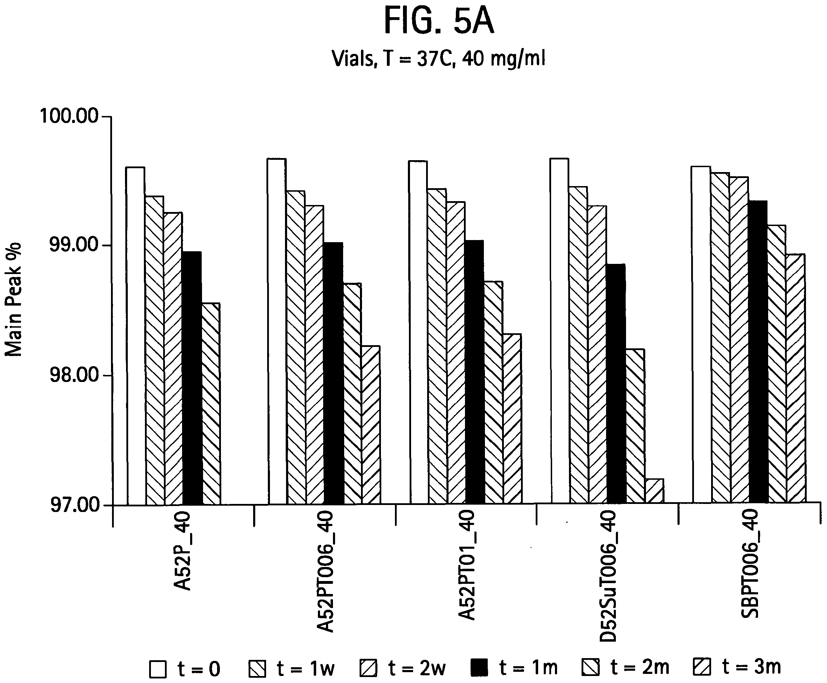

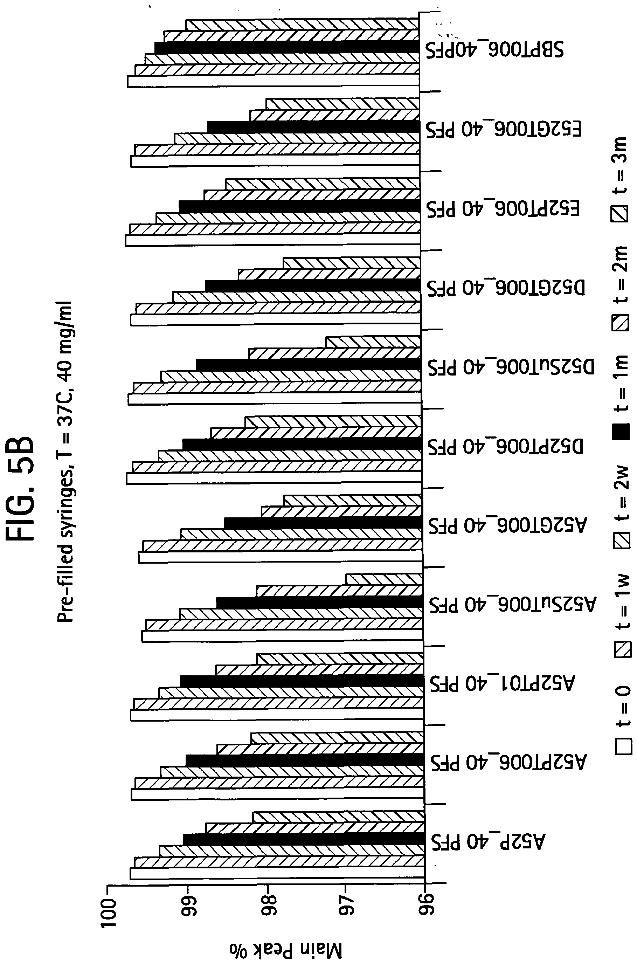

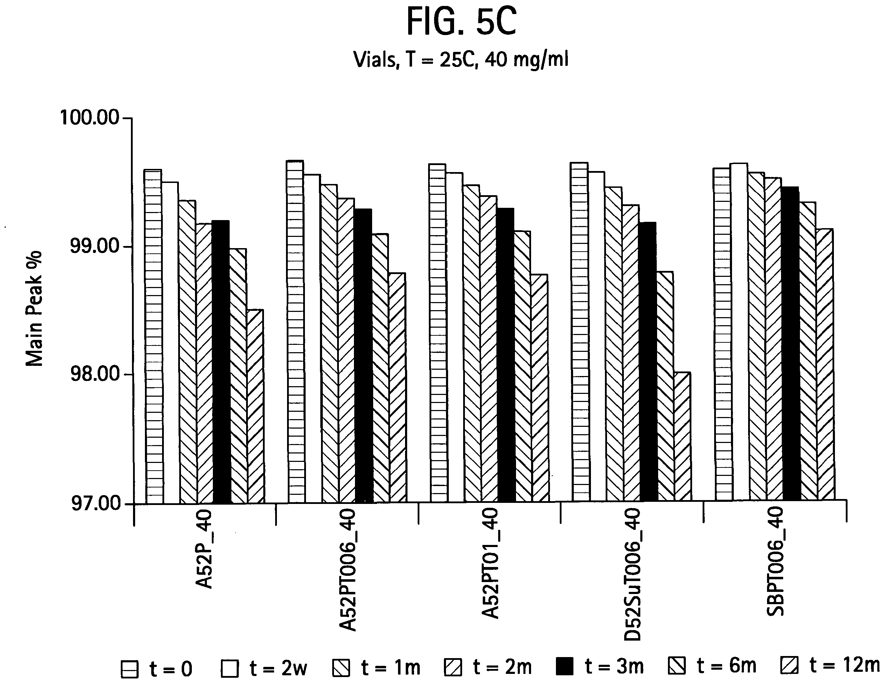

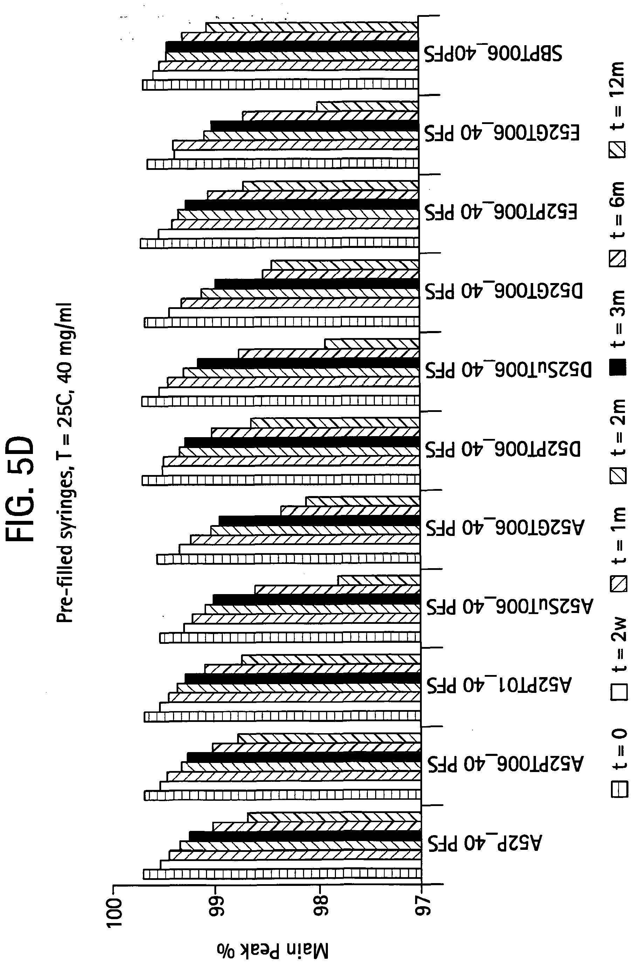

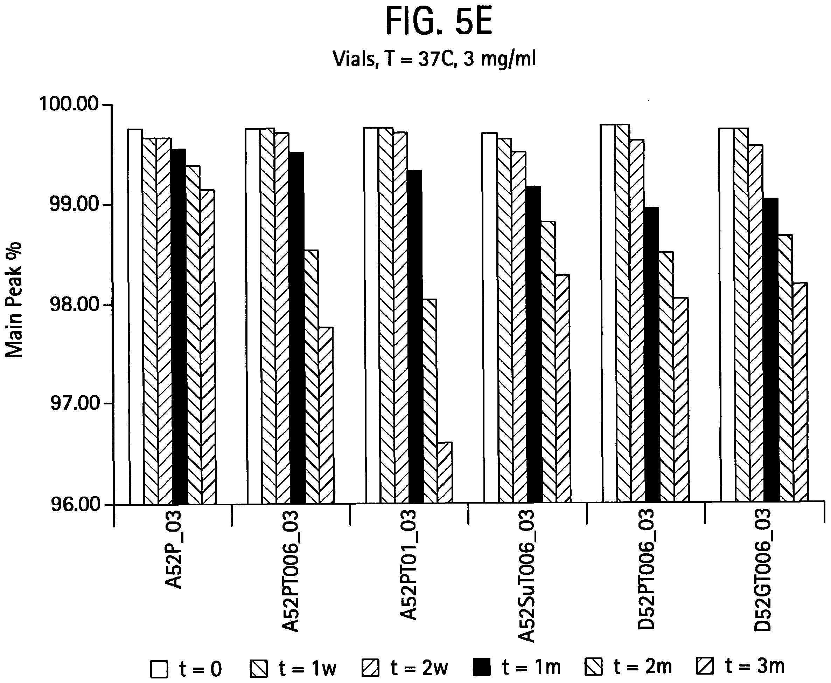

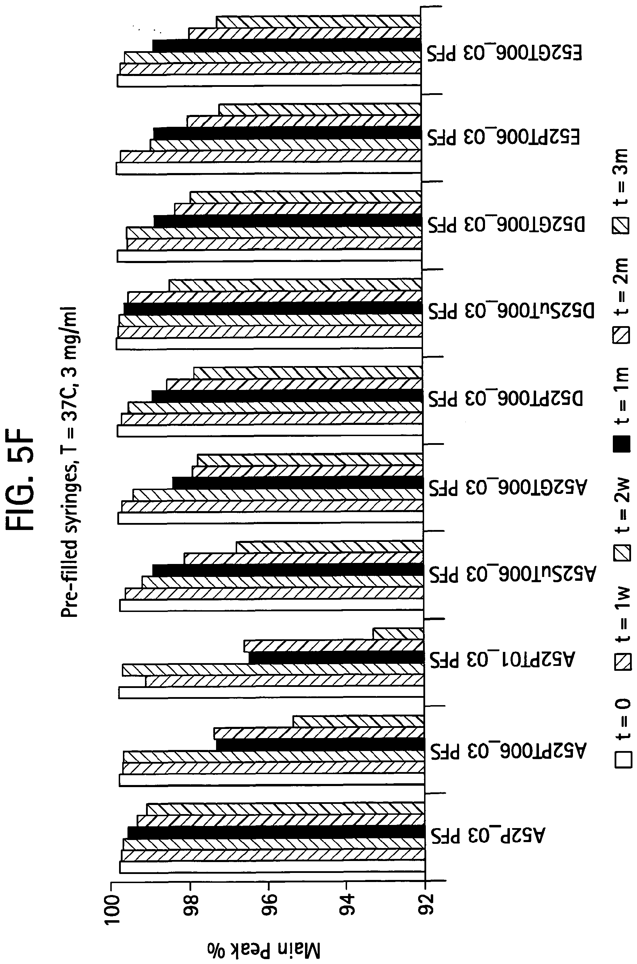

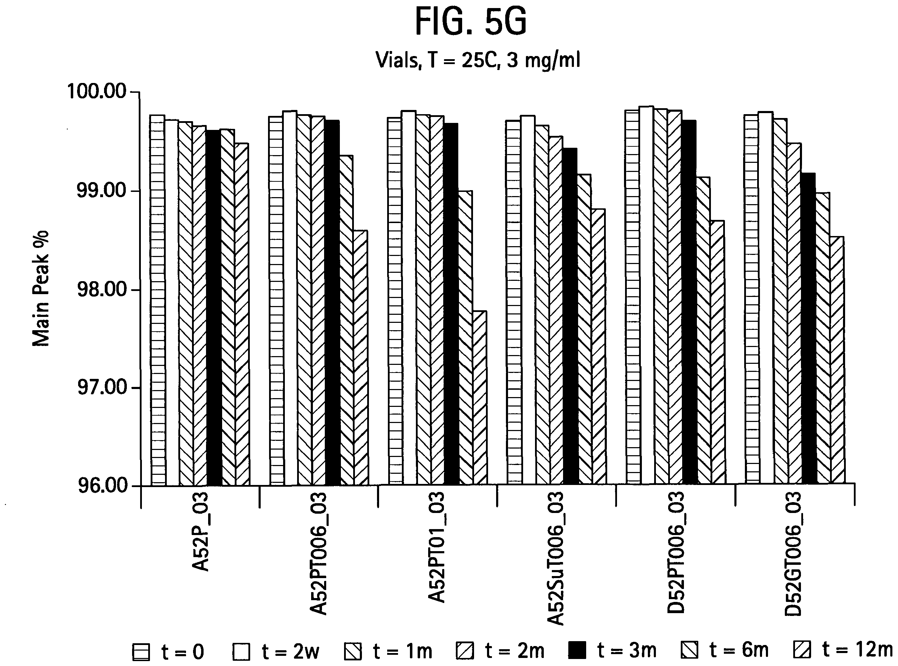

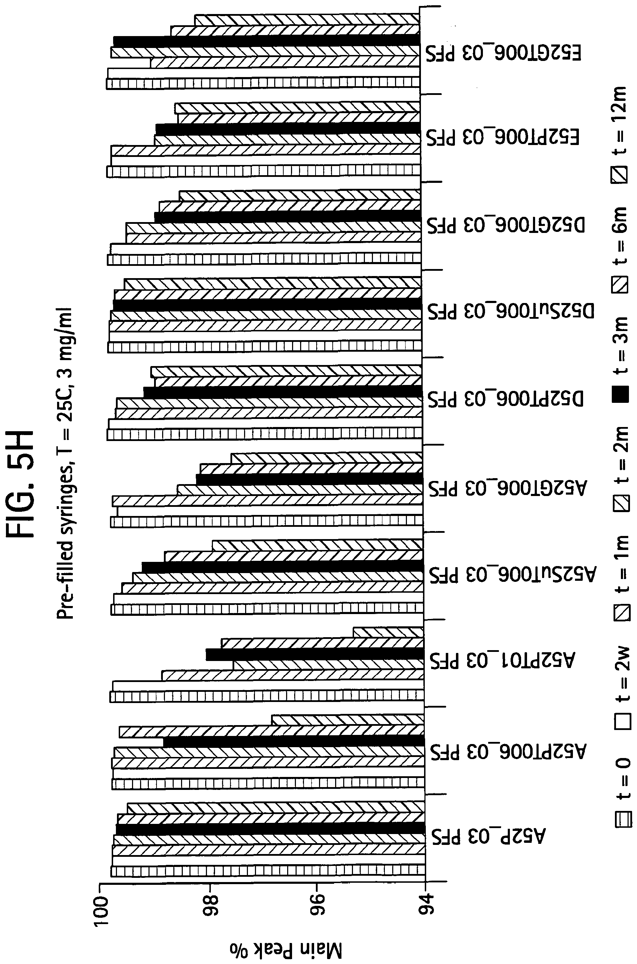

Figures 5A-5H illustrates the main peak percent areas obtained by size exclusion chromatography (SEC) for different formulations stored at 25°C or 37°C in glass vials and pre-filled syringes (PFS) at an antibody concentration of 40 mg/ml (Figures 5A-5D) or 3 mg/ml (Figures 5E-5H)

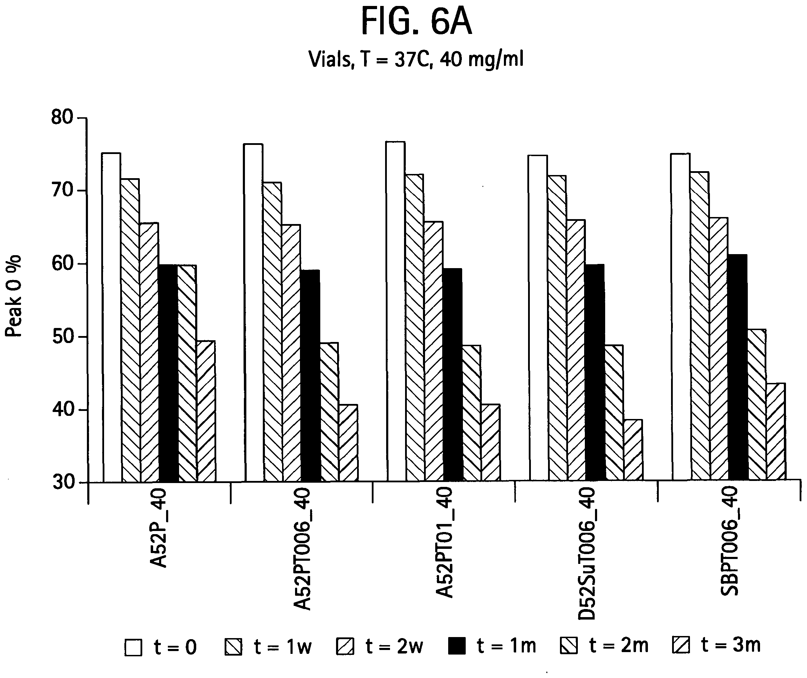

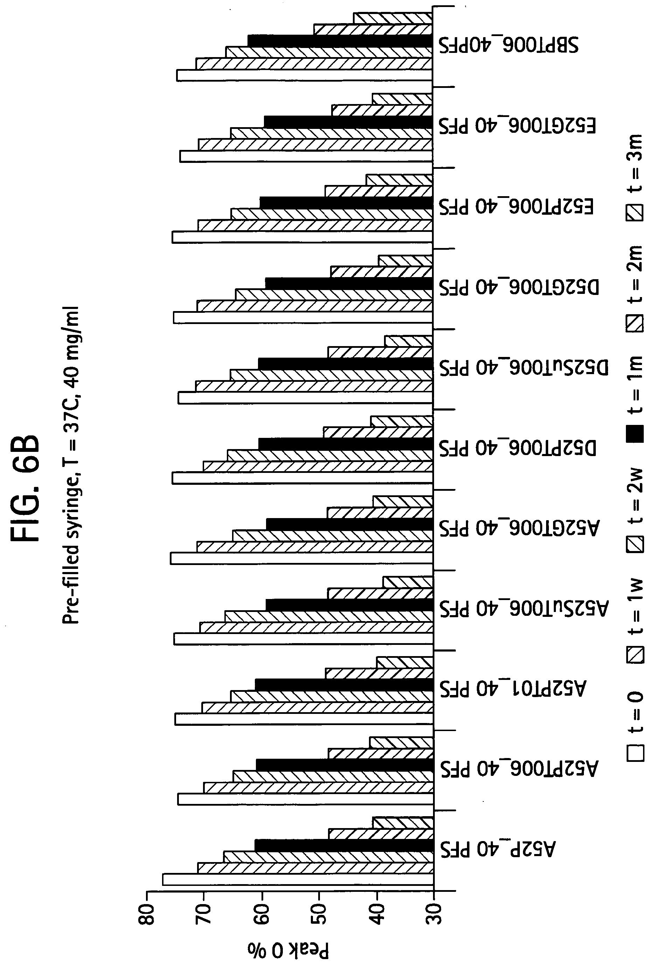

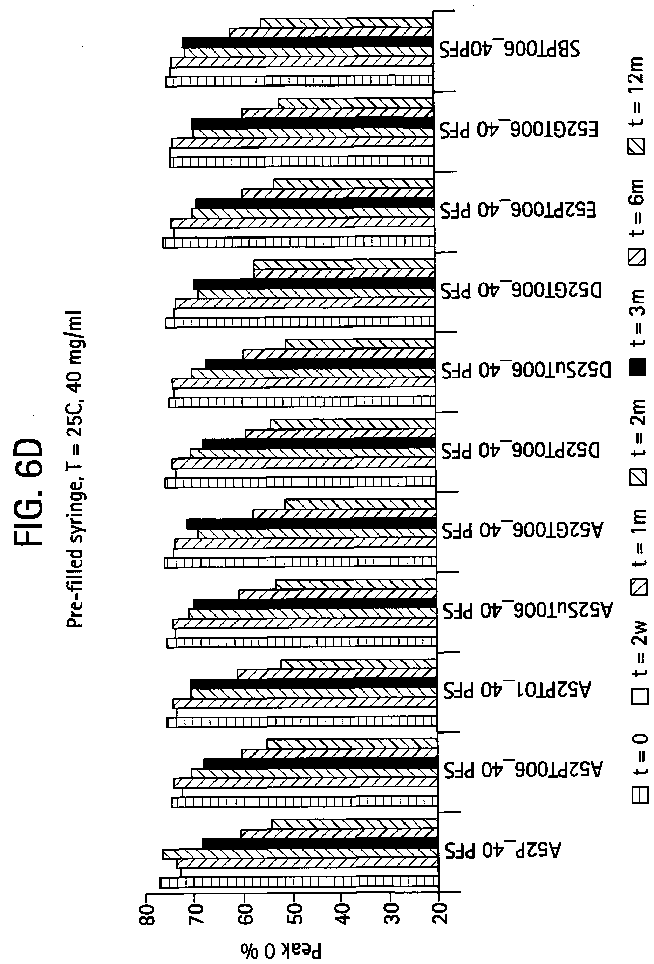

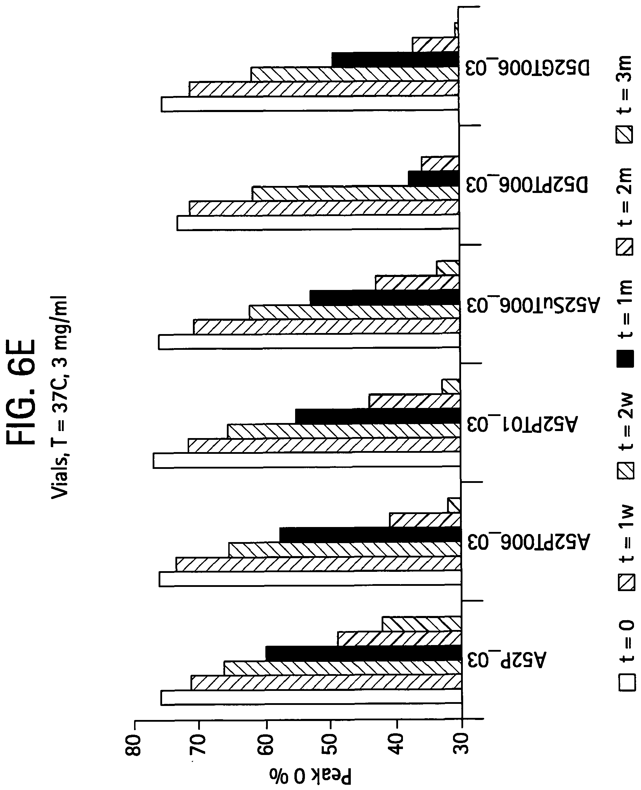

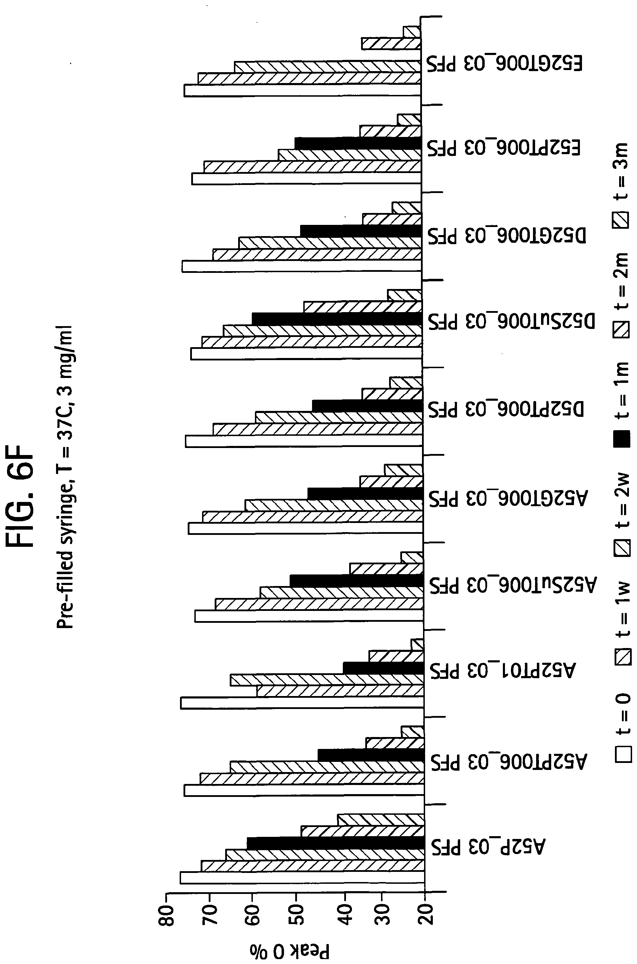

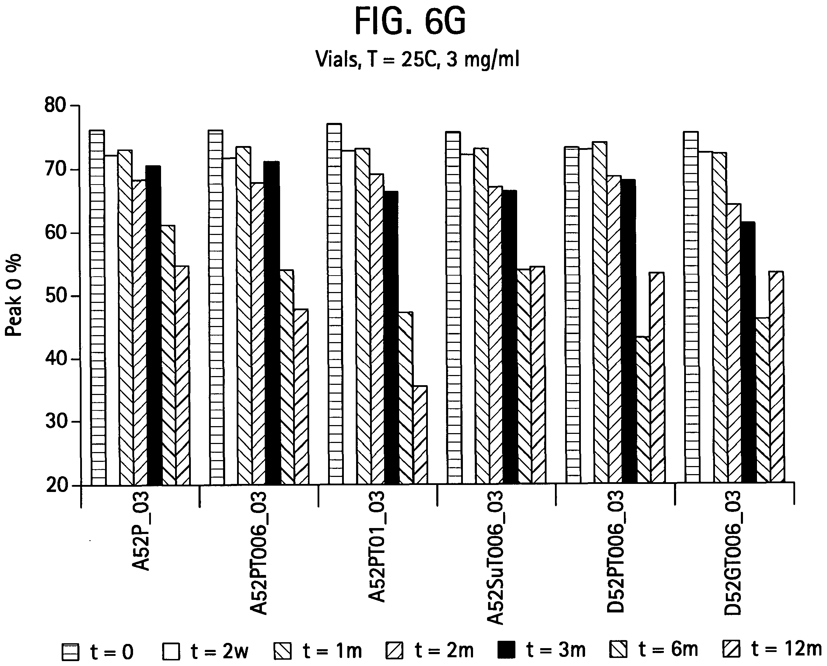

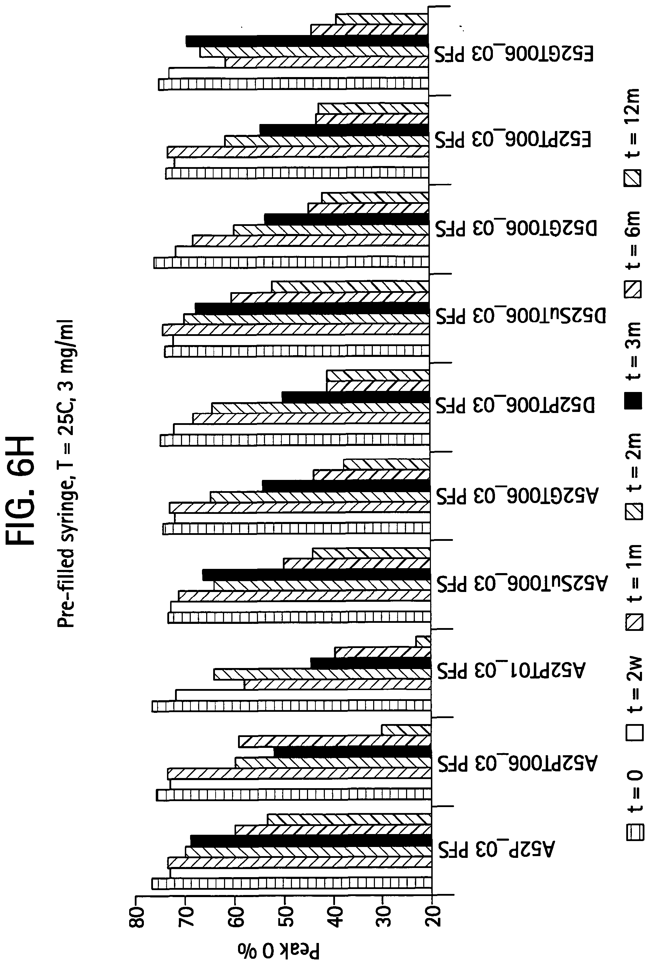

Figures 6A-6H illustrates cation exchange chromatography obtained for different formulations stored at 25°C or 37°C in glass vials and pre-filled syringes at an antibody concentration of 40 mg/ml (Figures 6A-6D) and 3 mg/ml (Figures 6E-6H).

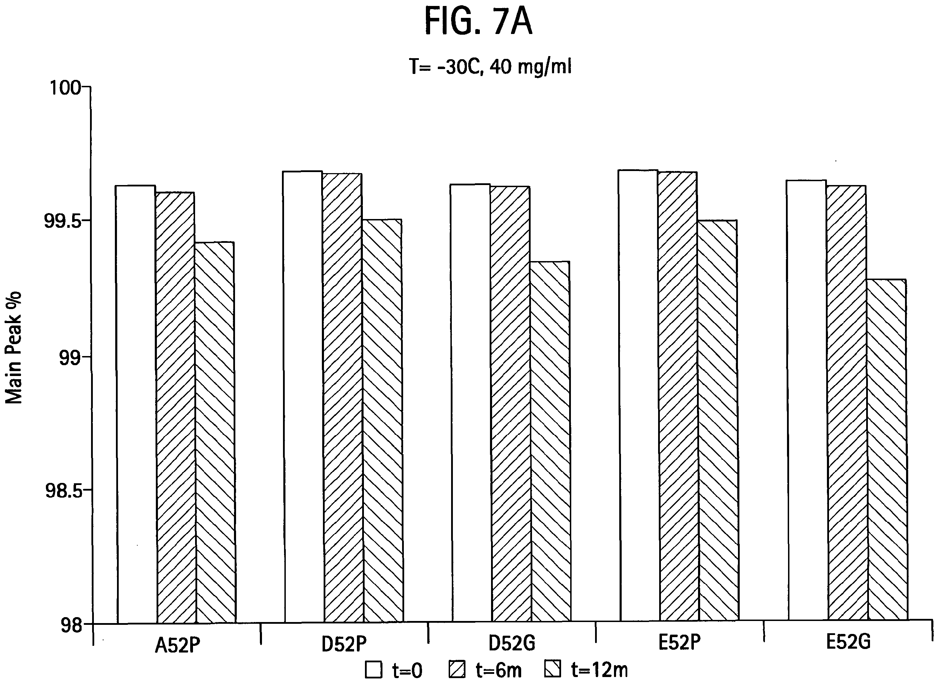

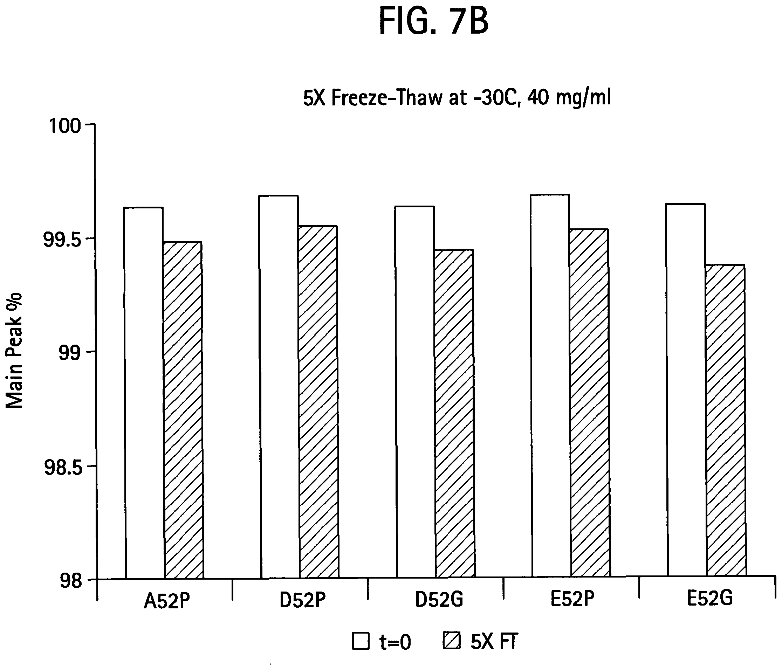

Figures 7A-7B. Figure 7A illustrates long-term stability of an antibody solution stored at -30°C. Figure 7B illustrates stability of an antibody following multiple freeze thaw cycles at -30°C .

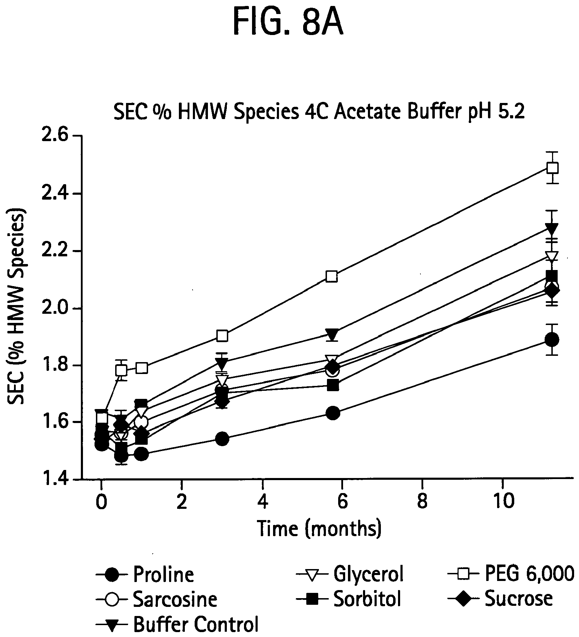

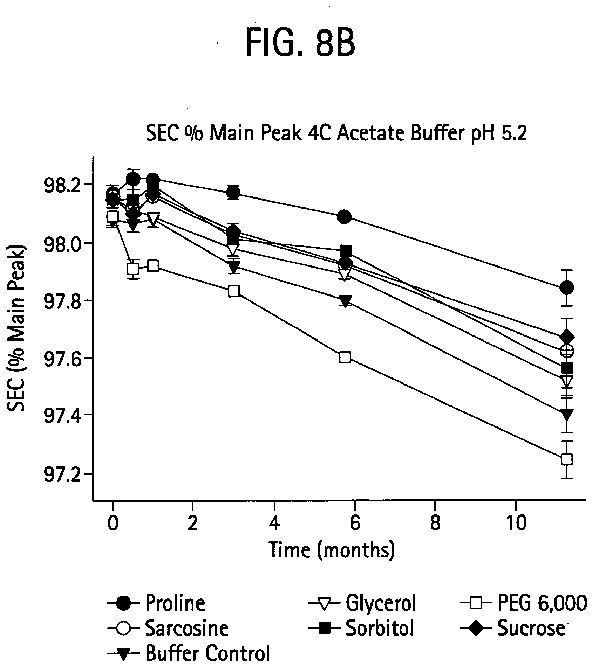

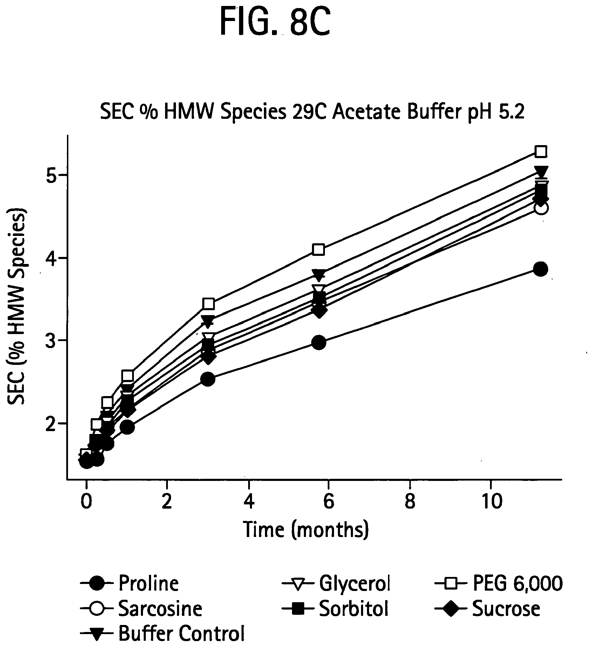

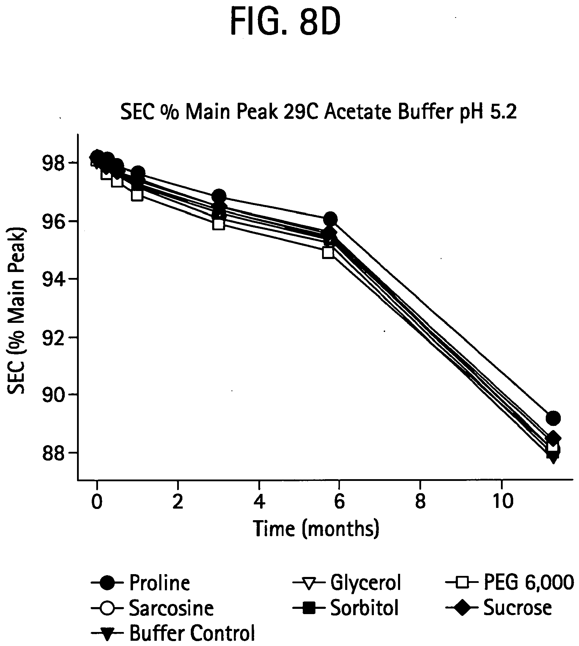

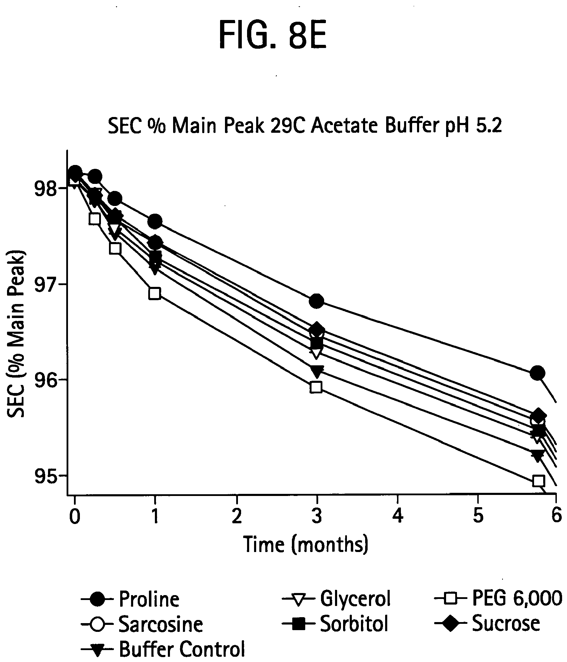

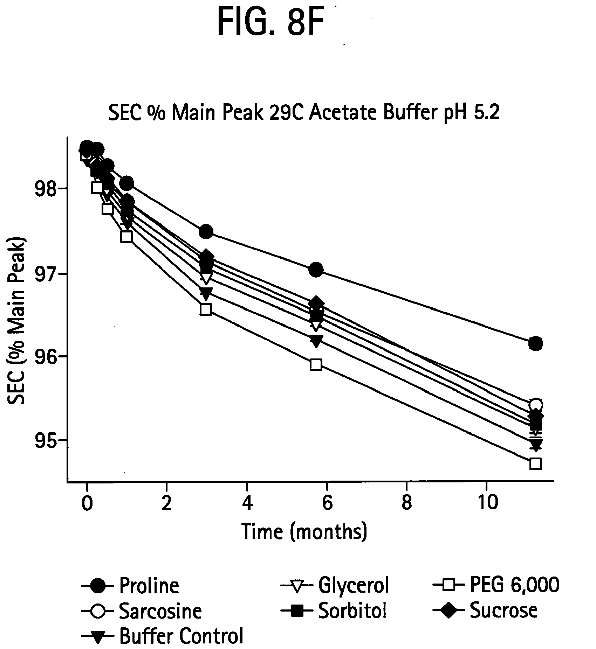

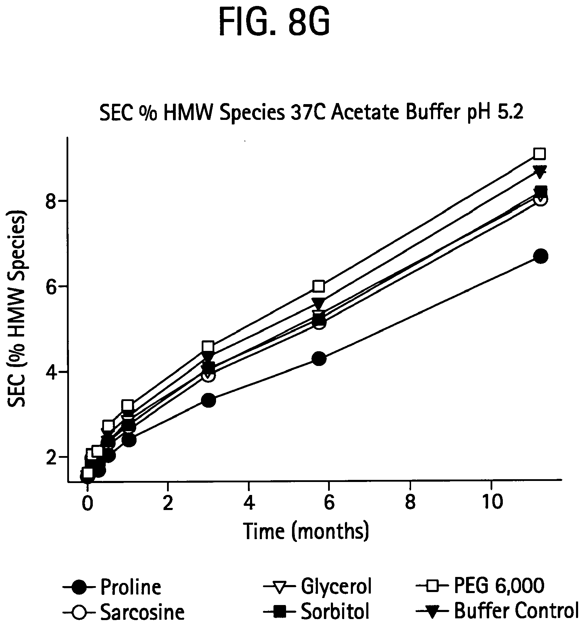

Figures 8A-8G provide results using SEC for different storage conditions using different formulations. The antibody was stored in a solution containing 100 mg/ml sodium acetate at pH 5.2. All excipients were at a concentration of 270 mM except PEG 6,000 which was at 2%. Results in Figures 8A, 8C and 8G are expressed in terms of HMW aggregates, while results in Figures 8B, 8D, 8E and 8F are expressed in terms of a percent of the main peak. In all instances, solutions containing proline provided the best results.

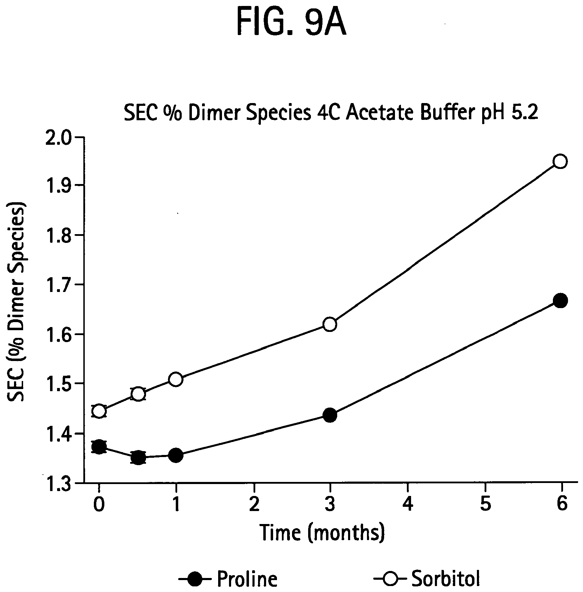

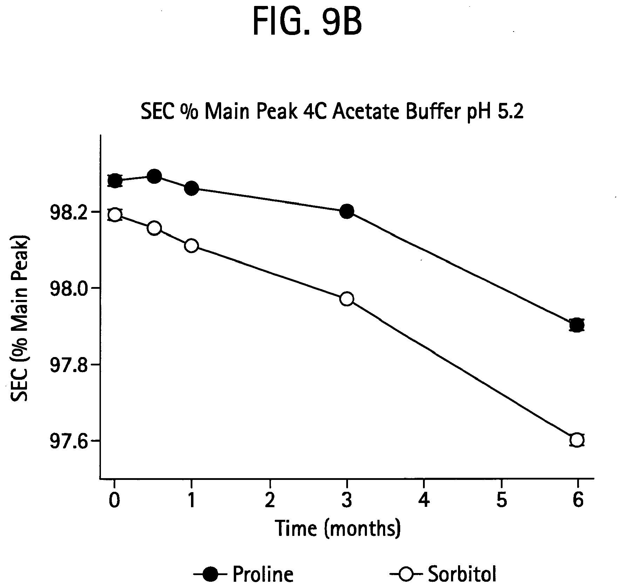

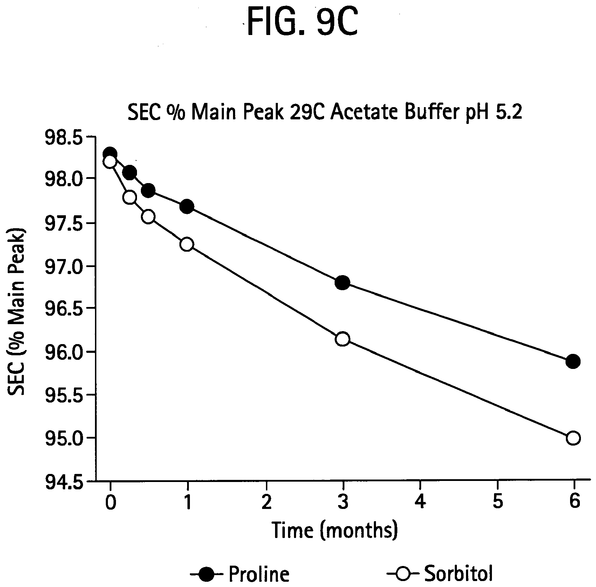

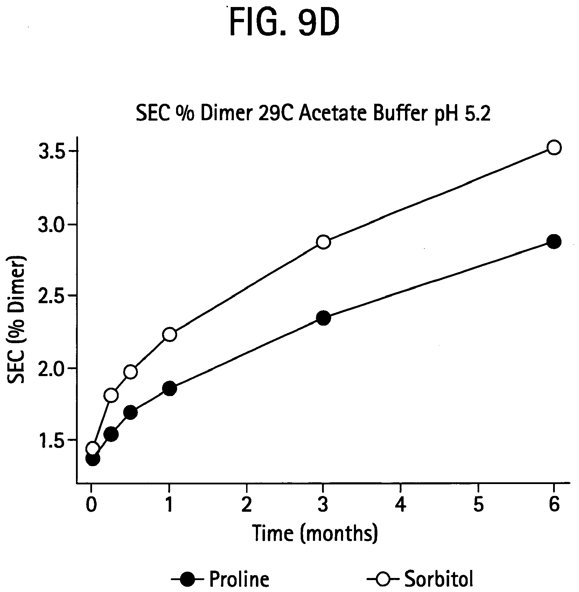

Figures 9A-9D illustrates results from use of polysorbate in the storage solution.

### DETAILED DESCRIPTION

Various embodiments in this specification are directed to a formulation that exhibits a stabilizing capacity for polypeptides. The formulation can comprise an acetic acid buffer, proline and a protein. The buffer has a pH from about 4.0 to about 6.0. The polypeptide can be an antibody. In various embodiments, the formulation can consist of or consist essentially of acetic acid buffer, proline and a protein. The formulation does not further comprise both a polyol and a surfactant.

Biopharmaceuticals included in the formulation can, in some instances, exhibit stability for long periods of time, for example, at least one or more months at various temperature, for example, approximately 4°C, 25°C or 37°C thereby allowing the administration of a safe and effective amount of a therapeutic polypeptide or other biopharmaceutical. In various embodiments, stability of antibodies can be demonstrated for 12-18 months at approximately 4°C.

The following definitions are provided to facilitate understanding of certain terms used throughout the specification.

It should be understood that while various embodiments in the specification are presented using "comprising" language, under various circumstances, a related embodiment may also be described using "consisting of' or "consisting essentially of' language.

It should also be understood that when describing a range of values, the characteristic being described could be an individual value found within the range. For example, "a pH from about pH 4 to about pH 6," could be pH 4, 4.2, 4.6, 5.1 5.5 etc. and any value in between such values. Additionally, "a pH from about pH 4 to about pH 6," should not be construed to mean that the pH of a formulation in question varies 2 pH units in the range from pH 4 to pH 6 during storage, but rather a value may be picked in that range for the pH of the solution, and the pH remains buffered at about that pH.

It is to be noted that the term "a" or "an", refers to one or more, for example, "an immunoglobulin molecule," is understood to represent one or more immunoglobulin molecules. As such, the terms "a" (or "an"), "one or more," and "at least one" can be used interchangeably herein.

As used herein, the term "about" means that a number referred to as "about" comprises the recited number plus or minus 5% of that recited number. For example, "about 50 mM" can mean 47.5, 47.6, 47.7, 47.8, 47.9, 48, 49, 50, 51, 52 or 52.5 mM or other values that are plus or minus 5% of 50, depending on the situation.

As used herein, the term "acetic acid buffer" is intended to mean a buffer comprising acetic acid. The buffer can be made from an acetate salt, for example, sodium acetate. Other salts can be used, for example, potassium, ammonium, calcium or magnesium salts of acetate. "Acetic acid buffer" and "acetate buffer" are used interchangeably.

As used herein, the term "biopharmaceutical" refers to a macromolecule or biopolymer, for example, a polypeptide or antibody, that can be used as a pharmaceutical.

As used herein, the term "formulation(s)" means a combination of at least one active ingredient with one or more other ingredients for one or more particular uses, such as storage, further processing, sale, and/or administration to a subject, such as, for example, administration to a subject of a specific agent in a specific amount, by a specific route, to treat a specific disease. The term "formulation", refers to a pharmaceutically acceptable medium that is compatible with a biopharmaceutical that can be administered to humans or animals.

As used herein, the term "effective amount" when used in reference to a therapeutic biopharmaceutical such as a therapeutic polypeptide is intended to mean an amount of the therapeutic molecule sufficient to ameliorate or mitigate at least one symptom associated with a targeted disease or physiological condition.

In various embodiments, the specification provides a formulation comprising proline, an effective amount of a therapeutic polypeptide and an aqueous solution having a pH from about 4.0 to about 6.0. The aqueous solution can be an aqueous buffer. The formulation can exhibit optimal properties for administration, storage and/or manipulation of biopharmaceuticals. Manipulation can include, for example, lyophilization, reconstitution, dilution, titration, and storage. The aqueous buffering component, i.e. an acetic acid buffer, can be combined with a desired biopharmaceutical using any of a variety of methods known in the art. Additionally, the buffering component can be compatible with a wide variety of excipients and surfactants that facilitate stability of a biopharmaceutical of interest. These and other attributes of the formulation can allow stable formulations of bioactive molecules to be prepared and maintained over prolonged periods.

As used herein, the term "excipient" is intended to mean a therapeutically inactive substance. Excipients can be included in a formulation for a wide variety of purposes including, for example, as a diluent, vehicle, buffer, stabilizer, tonicity agent, bulking agent, surfactant, cryoprotectant, lyoprotectant, anti-oxidant, metal ion source, chelating agent and/or preservative. Excipients include, for example, polyols such as sorbitol or mannitol; sugars such as sucrose, lactose or dextrose; polymers such as polyethylene glycol; salts such as NaCl, KC1 or calcium phosphate, amino acids, for example, proline, glycine or methionine, surfactants, metal ions, buffer salts such as glutamate, acetate or aspartate, preservatives and polypeptides such as human serum albumin, as well as saline and water. Excipients can comprise sugars, for example sugar alcohols, reducing sugars, non-reducing sugars and sugar acids. Excipients are well known in the art and can be found described in, for example, Wang W., Int. J. Pharm. 185:129-88 (1999) and Wang W., Int. J. Pharm. 203:1-60 (2000).

Briefly, sugar alcohols, also known as a polyols, polyhydric alcohols, or polyalcohols, are hydrogenated forms of carbohydrate having a carbonyl group reduced to a primary or secondary hydroxyl group. Polyols can be used as stabilizing excipients and/or isotonicity agents in both liquid and lyophilized formulations. Polyols can protect biopharmaceuticals from both physical and chemical degradation pathways. Examples of sugar alcohols can include sorbitol, glycerol, mannitol, xylitol, maltitol, lactitol, erythritol and threitol.

Reducing sugars can comprise, for example, sugars with a ketone or aldehyde group and contain a reactive hemiacetal group, which allows the sugar to act as a reducing agent. Specific examples of reducing sugars include fructose, glucose, glyceraldehyde, lactose, arabinose, mannose, xylose, ribose, rhamnose, galactose and maltose. Non-reducing sugars can comprise an anomeric carbon that is an acetal and is not substantially reactive with amino acids or polypeptides to initiate a Maillard reaction. Specific examples of non-reducing sugars include sucrose, trehalose, sorbose, sucralose, melezitose and raffinose. Sugar acids include, for example, saccharic acids, gluconate and other polyhydroxy sugars and salts thereof.

Buffers or buffers in combination with excipients can maintain the pH of liquid formulations throughout product shelf-life and maintain the pH of lyophilized formulations during the lyophilization process and upon reconstitution, for example.

Tonicity agents and/or stabilizers included in liquid formulations can be used, for example, to provide isotonicity, hypotonicity or hypertonicity to a formulation such that it is suitable for administration. Such excipients also can be used to facilitate maintenance of a biopharmaceuticals' structure and/or to minimize electrostatic, solution protein-protein interactions. Examples of tonicity agents and/or stabilizers can include polyols, salts and/or amino acids.

Anti-oxidants are useful in liquid formulations to control protein oxidation and also can be used in lyophilized formulations to retard oxidation reactions.

Metal ions can be included in a liquid formulation, for example, as a co-factor and divalent cations such as zinc and magnesium can be utilized in suspension formulations. Chelating agents included in liquid formulations can be used, for example, to inhibit metal ion catalyzed reactions. With respect to lyophilized formulations, metal ions also can be included, for example, as a co-factor. Although chelating agents are generally omitted from lyophilized formulations, they also can be included as desired to reduce catalytic reactions during the lyophilization process and upon reconstitution.

Preservatives in liquid formulations can be used, for example, to protect against microbial growth and are particularly beneficial in multi-dose formulations. In lyophilized formulations, preservatives are generally included in the reconstitution diluent. Benzyl alcohol is a specific example of a preservative useful in a formulation of the invention.

As used herein, the term "surfactant" is intended to mean a substance that functions to reduce the surface tension of a liquid in which it is dissolved. Surfactants can be included in a formulation for a variety of purposes including, for example, to prevent or control aggregation, particle formation and/or surface adsorption in liquid formulations or to prevent or control these phenomena during the lyophilization and/or reconstitution process in lyophilized formulations. Surfactants include, for example, amphipathic organic compounds that exhibit partial solubility in both organic solvents and aqueous solutions. General characteristics of surfactants include their ability to reduce the surface tension of water, reduce the interfacial tension between oil and water and also form micelles. Surfactants of can include non-ionic and ionic surfactants. Surfactants are known in the art and can be found described in, for example, Randolph T.W. and Jones L.S., Surfactant-protein interactions. Pharm Biotechnol. 13:159-75 (2002).

Non-ionic surfactants can include, for example, alkyl poly (ethylene oxide), alkyl polyglucosides such as octyl glucoside and decyl maltoside, fatty alcohols such as cetyl alcohol and oleyl alcohol, cocamide MEA, cocamide DEA, and cocamide TEA. Specific examples of non-ionic surfactants include the polysorbates including, for example, polysorbate 20, polysorbate 28, polysorbate 40, polysorbate 60, polysorbate 65, polysorbate 80, polysorbate 81, and polysorbate 85; the poloxamers including, for example, poloxamer 188, also known as poloxalkol or poly(ethylene oxide)-poly(propylene oxide), poloxamer 407 or polyethylene-polypropylene glycol, and polyethylene glycol (PEG). Polysorbate 20 is synonymous with TWEEN 20, sorbitan monolaurate and polyoxyethylenesorbitan monolaurate.

Ionic surfactants can include, for example, anionic, cationic and zwitterionic surfactants. Anionic surfactants include, for example, sulfonate-based or carboxylate-based surfactants such as soaps, fatty acid salts, sodium dodecyl sulfate (SDS), ammonium lauryl sulfate and other alkyl sulfate salts. Cationic surfactants include, for example, quaternary ammonium-based surfactants such as cetyl trimethylammonium bromide (CTAB), other alkyltrimethylammonium salts, cetyl pyridinium chloride, polyethoxylated tallow amine (POEA) and benzalkonium chloride. Zwitterionic or amphoteric surfactants include, for example, dodecyl betaine, dodecyl dimethylamine oxide, cocamidopropyl betaine and coco ampho glycinate.

As used herein, the term "therapeutic" when used in reference to a polypeptide, e.g. an antibody, is intended to mean that the polypeptide can be used in the cure, mitigation, treatment or prevention of disease in a human or other animal. Treatment refers to both therapeutic treatment and/or prophylactic or preventative measures. Those in need of treatment can include those already with a disorder or those in which a disorder is to be prevented.

Accordingly, a therapeutic polypeptide can be a biopharmaceutical and can comprise a single polypeptide or two or more polypeptide subunits. A therapeutic polypeptide can comprise an antibody (e.g. a "therapeutic antibody"), a functional antibody fragment thereof, an antigen-binding fragment, a peptibody or functional fragment thereof, growth factors, cytokines, cell signaling molecules and hormones. A wide variety of therapeutic polypeptides are known in the art, and are included within the meaning of the term "therapeutic polypeptides" as it is used herein. Therapeutic polypeptides to be used in the formulation described in this specification can comprise, for example, antibodies or antigen-binding fragments, to a wide variety of antigens, for example, interleukins, G-CSF, GM-CSF, kinases, TNF and TNFR, RANKL, EGFR, ligands, cyclins and erythropoietin and or growth factors. Growth factors can comprise, for example, epidermal growth factor, human growth factor or nerve growth factor.

Stability of a formulation refers to the retention of structure and/or function and/or biological activity of a biopharmaceutical within the formulation. The retention of structure and/or function and/or biological activity does not need to be 100%. Measurement of the stability of a formulation can be a comparative measure. Therefore, if one formulation is said to be more stabile or have greater stability than another, the formulation with greater stability has retained a greater percentage of a desired characteristic being investigated than the other formulation, unless the characteristic being considered is a negative characteristic. If the characteristic is a negative characteristic, then the formulation with greater stability will have less of that characteristic. For example, formulation A is more stable than formulation B if it maintains a greater percentage of the main peak when measured by size exclusion chromatography, i.e. it demonstrates less aggregation. Formulation A can also be said to be more stable than formulation B if it contains fewer particles than formulation B following storage.

In various embodiments, a biopharmaceutical in the formulation can exhibit attributes such as resistance to change or deterioration that affect stability or function and therefore maintain consistent functional characteristics over time.

In various embodiments, the stability of a biopharmaceutical within a formulation can comprise, the retention of physical and/or chemical stability. Biopharmaceutical stability can be assessed by, for example, determining whether the biopharmaceutical has been subjected to a physical degradation and/or chemical degradation pathway, including chemical modification of its structure. Retention of stability can also be measured, for example, in terms of the percentage of monomer remaining, after storage at different temperatures or after multiple freeze-thaw cycles. These measurements can reflect the amount of polypeptide aggregation.

Retention of physical or chemical stability can be determined, by measurements of the percentage of monomer before and after storage, for example, by size exclusion chromatography (SEC). In various embodiments, the percentage of monomer remaining after storage or repeated freeze-thawing can be between about 80% and about 100%, between about 85% and about 95%, or between about 90% and about 95%, or between about 95% and about 99% when compared to the biopharmaceutical at an initial time point. Accordingly, stability of a biopharmaceutical within a formulation of the invention can include retention of stability greater than 99.5%, at least 99%, 98%, 97%, 96%, 95%, 94%, 93%, 92%, 91%, 90%, 89%, 88%, 87%, 86%, 85%, 84%, 83%, 82%, 81% or 80% compared to the stability of the biopharmaceutical at an initial time point.

Other examples of the stability of formulations can comprise comparative measurements of the number of insoluble proteinaceous aggregates (particles) in solution, or occurrence of chemical modifications relative to the biopharmaceutical in the starting solution.

In additional embodiments, stability of a formulation includes, for example, retention of activity. Biopharmaceutical activity can be assessed using, for example, an *in vitro, in vivo* and/or *in situ* assay indicative of the biopharmaceutical's function. Retention of stability of a biopharmaceutical in a formulation of the invention can include, for example, retention of activity between about 50 and about 100% or more, depending on the variability of the assay. For example, retention in stability can include retention of activity between about 60% and about 99% or between about 70% and about 80% compared to the activity of the biopharmaceutical at an initial time point.

In various embodiments, stability of a biopharmaceutical within a formulation can include retention of activity of at least 50%, 55%, 60%, 65%, 70%, 75%, 80%, 85%, 90%, 95% or 100% and can include activity measurements greater than 100%, for example, 105%, 110%, 115%, 120%, 125% or 150% or more compared to the activity of the biopharmaceutical at an initial time point.

Generally, an initial time point is selected to be the time that a biopharmaceutical is first prepared in a formulation or first examined for quality (for example, meets release specifications), in various embodiments. An initial time point can also include the time at which a biopharmaceutical is reformulated in a formulation. The reformulation can be, for example, at a higher concentration, lower concentration or at the same concentration of an initial preparation.

Stability of a biopharmaceutical in a formulation can be retained at 2-8°C or at approximately 4°C. The stability of a biopharmaceutical in a formulation as described herein can also be retained at temperatures above 4°C, for example, at room temperature, about 23°C or 25°C, or higher, including 37°C. This greater retention in stability at higher temperatures can be shown by the greater retention of the main peak of, for example, an antibody in a glutamic acid buffered formulation comprising proline as shown in Figure 1 (reference example) compared to other buffers without proline.

According to various embodiments, the formulation described in the specification is stable at about 4°C, about 25°C or about 37°C for at least six months, such that the monomer peak can represent at least 99% of the total as determined by SEC. In various other embodiments, the monomer peak represents at least 99.8%, 99.7%, 99.6%, 99.5%, 98%, 97%, 96%, 95%, 94%, 93%, 92%, 91% or 90% of the total as determined, by example, using size exclusion chromatography. In various other embodiments, the formulation can be stable for at least one month, at least two months, at least three months, at least four months, at least five months or more than about six months.

A formulation can be prepared to be isotonic with a reference solution or fluid (for example, blood and/or serum). An isotonic solution has a concentration such that it is osmotically stable. Unless expressly compared to a specific solution or fluid, isotonic or isotonicity as used herein is intended to mean reference to human blood serum (e.g., 280-300 mOsm/kg). Therefore, an isotonic formulation will contain a substantially similar concentration of solutes or exhibit substantially similar osmotic pressure as human blood. In general, an isotonic solution contains about the same concentration of solutes as normal saline for humans and many other mammals, which is about 0.9 weight percent (0.009 g/ml) salt in aqueous solution (e.g., 0.009 g/ml NaCl).

Many aspects of pharmaceutical production and formulation processes can be pH sensitive. Maintaining the correct pH of a finished pharmaceutical product can effect the pharmaceutical's stability, effectiveness, and shelf life, and pH is an consideration in designing formulations for administration that will be acceptable, as well as safe and effective.

To maintain pH, pharmaceutical processes and formulations can use one or more buffering agents. A variety of buffering agents are available for pharmaceutical use. The buffer or buffers for a given application should be effective at the desired pH. They should also provide sufficient buffer capacity to maintain the desired pH for as long as necessary. A good buffer for a pharmaceutical composition can satisfy numerous other requirements as well. It should be appropriately soluble. It should not form deleterious complexes with metal ions, be toxic, or unduly penetrate, solubilize, or absorb on membranes or other surfaces. It should not interact with other components of the composition in any manner which decreases their availability or effectiveness. It should be stable and effective at maintaining pH over the range of conditions to which it will be exposed during formulation and during storage of the product. It should not be deleteriously affected by oxidation or other reactions occurring in its environment, such as those that occur in the processing of the composition in which it is providing the buffering action. If carried over or incorporated into a final product, a buffering agent should be safe for administration, compatible with other components of the composition over the shelf-life of the product, and acceptable for administration to the end user. The above list represents various characteristics related to a formulation containing a biopharmaceutical. Not all buffers, however, will necessarily exhibit all of the described characteristics.

In various embodiments, the formulations described herein can be approved for pharmaceutical use by a national or international authority empowered by law to grant such approval for example, the European Agency for the Evaluation of Medical Products, Japan's Ministry of Health, Labor and Welfare, China's State Drug Administration, United States Food and Drug Administration, or their successor(s) in this authority, particularly preferably the United States Food and Drug Administration or its successor(s) in this authority.

The formulation contains acetic acid (acetate) as a buffer with proline and a biopharmaceutical. The formulation can contain a concentration of buffer having sufficient buffering capacity to maintain a selected pH of the formulation at a selected temperature. Useful concentrations of a buffer can be between about 1 mM-150 mM, between about 5 mM-100 mM, between about 10 mM-50 mM or between about 20 to about 40 mM. In various embodiments, the buffer concentration can be about 5 mM, about 6 mM, about 7 mM, about 8 mM, about 9 mM, about 10 mM, about 11 mM, about 12 mM, about 13 mM, about 14 mM, about 15 mM, about 20 mM, about 25 mM, about 30 mM, about 35 mM, about 40 mM, or about 45 mM. Other concentrations of buffer can be appropriate provided that the buffer has sufficient buffering capacity to maintain a selected pH of a formulation at a selected temperature during storage.

In various other embodiments, the concentration of the buffering solution can be from about 1 mM to about 100 mM, from about 2 mM to about 50 mM, from about 3mM to about 30 mM, from about 4 mM to about 20 mM, or from about 5 mM to about 10 mM, from about 10 mM to about 40 mM, from about 15 mM to about 35 mM, from about 20 mM to about 30 mM, from about 25 mM to about 35 mM, about 26 mM, about 27 mM, about 28 mM, about 29 mM, about 30 mM, about 31 mM about 32 mM, about 33 mM or about 34 mM.

In various embodiments, the formulation comprises proline at a concentration of about 2%, about 3%, about 4%, about 5%, about 6%, about 7%, about 8% about 9%, about 10%.

In various embodiments, the buffer of a formulation can include one or more excipients. One potential role of an included excipient is to provide stabilization of the biopharmaceutical against stresses that can occur during manufacturing, shipping and storage. To accomplish this, at least one excipient can function as a buffer, stabilizer, tonicity agent, bulking agent, surfactant, cryoprotectant, lyoprotectant, anti-oxidant, metal ion source, chelating agent and/or preservative. In addition, at least one excipient can function as a diluent and/or vehicle or be employed to reduce viscosity in high concentration formulations in order to enable their delivery and/or enhance patient convenience.

Similarly, at least one excipient can confer more than one of the above functions onto a formulation. Alternatively, two or more excipients can be included in a formulation to perform more than one of the above or other functions. For example, an excipient can be included as a component in a formulation to change, adjust or optimize the osmolality of the formulation, thereby acting as a tonicifier.

In various embodiments, a tonicity agent and a surfactant can both be included in a formulation to both adjust the osmolality and/or control aggregation. Excipients, their use, formulation and characteristics are known in the art and can be found described in, for example, Wang W., Int. J. Pharm. 185:129-88 (1999) and Wang W., Int. J. Pharm. 203:1-60 (2000). In US Patent No. 6,171,586 B1 a formulation comprising an acetate buffer, a polyol, surfactant and antibody is discussed.

Small organic molecules referred to as osmolytes have been reported to affect protein stability in various physiological conditions. In this regard, a publication has discussed proline as a natural osmolyte and its effect on protein aggregation *in vivo* and *in vitro* (Ignatova and Gierasch, Proc. Natl. Acad. Sci. USA 103:13357-13361, Epub 2006 Aug 9). Bolen and Baskakov (J. Mol. Biol. 310:955-963, 2001) has also discussed proline and osmolytes.

In general, excipients can be chosen on the basis of the mechanisms by which they stabilize proteins against various chemical and physical stresses. Certain excipients can be beneficial to include to alleviate the effects of a specific stress or to regulate a particular susceptibility of a specific biopharmaceutical. Other excipients can be beneficial to include because they can have more general effects on the physical and covalent stabilities of proteins. Some useful excipients can include those chemically and functionally innocuous or compatible with aqueous buffer solutions and biopharmaceuticals so as to optimize the stability properties of a formulation. Various such excipients are described herein as exhibiting chemical compatibility with the formulations and functional compatibility with the biopharmaceutical included in such formulations.

For example, excipients chosen to enhance or confer stability of a biopharmaceutical within a formulation can include those that are substantially free from reacting with functional groups on the biopharmaceutical. In this regard, both reducing and non-reducing sugars can be used as an excipient in a formulation of the invention.

Excipients can also be chosen to enhance or provide stabilization with reference to the mode of administration for a formulation. For example, parenteral routes of intravenous (IV), subcutaneous (SC) or intramuscular (IM) administration can be more safe and efficacious when all components of the formulation maintain physical and chemical stability during manufacture, storage and administration. Those skilled in the art can determine how to employ one or more excipients that maintain maximal stability of the active form of a biopharmaceutical given, for example, a particular manufacturing or storage condition or a particular mode of administration.

The amount or concentration of excipient to use in a formulation can vary depending on, for example, the amount of biopharmaceutical included in the formulation, the amount of other excipients included in the desired formulation, whether a diluent is desired or needed, the amount or volume of other components of the formulation, the total amount of components within a formulation, the specific activity of the biopharmaceutical and the desired tonicity or osmolality to be achieved. In various embodiments, different types of excipients can be combined into a single formulation. Accordingly, a formulation can contain a single excipient, two, three or four or more different types of excipients. Combinations of excipients can be useful in conjunction with a formulation that contains two or more different biopharmaceuticals. The excipients can exhibit similar or different chemical properties.

Given the teachings and guidance provided herein, those skilled in the art can determine what amount or range of excipient can be included in any particular formulation to achieve a formulation that promotes retention in stability of the biopharmaceutical. For example, the amount and type of a salt to be included in a formulation can be selected based on the desired osmolality (i.e., isotonic, hypotonic or hypertonic) of the final solution, as well as the amounts and osmolality of other components to be included in the formulation. Similarly, with reference to the type of polyol or sugar included in a formulation, the amount of such an excipient can depend on its osmolality. Inclusion of about 5% sorbitol can achieve isotonicity while about 9% of a sucrose excipient may be needed to achieve isotonicity. Those skilled in the art will understand that the considerations described herein concerning excipients can be equally applicable to all types and combinations of excipients including, for example, salts, amino acids, other tonicity agents, surfactants, stabilizers, bulking agents, cryoprotectants, lyoprotectants, anti-oxidants, metal ions, chelating agents and/or preservatives.

Excipients can be included in a formulation of the invention at concentration ranges generally between about 1-40% (w/v), between about 5-35% (w/v), between about 10-30% (w/v), between about 15-25% (w/v), about 3%, about 10% or about 20% (w/v). Concentrations as high as about 45% (w/v), 50% (w/v) or more than 50% (w/v) in certain instances can be employed in the formulations of the invention. For example, in some instances, it can be desirable to include concentrations up to 60% (w/v) or 75% (w/v) to produce a hypertonic formulation. Such hypertonic solutions can be diluted to produce an isotonic formulation prior to use if desired. Other useful concentration ranges include between about 1-20%, particularly between about 2-18% (w/v), more particularly between about 4-16% (w/v), even more particularly between about 6-14% (w/v) or between about 8-12% (w/v) or about 10% (w/v). In various embodiments, excipient concentrations and/or amounts less than, greater than or in between these ranges also can be used in a formulation. For example, one or more excipients can be included in a formulation which constitute less than about 1% (w/v). Similarly, a formulation can contain a concentration of one or more excipients greater than about 40% (w/v). Accordingly, a formulation can be produced that contains a desired concentration or amount of one or more excipients including, for example, 1, 2, 3, 4, 5, 6, 7, 8, 9, 10, 11, 12, 13, 14, 15, 16, 17, 18, 19 or 20% (w/v) or more.

Various excipients that can be useful in either a liquid or lyophilized formulation comprise, fucose, cellobiose, maltotriose, melibiose, octulose, ribose, xylitol, arginine, histidine, glycine, alanine, methionine, glutamic acid, lysine, imidazole, glycylglycine, mannosylglycerate, Triton X-100, Pluoronic F-127, cellulose, cyclodextrin, dextran (10, 40 and/or 70 kD), polydextrose, maltodextrin, ficoll, gelatin, hydroxypropylmeth, sodium phosphate, potassium phosphate, ZnCl₂, zinc, zinc oxide, sodium citrate, trisodium citrate, tromethamine, copper, fibronectin, heparin, human serum albumin, protamine, glycerin, glycerol, EDTA, metacresol, benzyl alcohol and phenol. Various excipients known in the art are described in, for example, Wang W., Int. J. Pharm. 185:129-88 (1999) and Wang W., Int. J. Pharm. 203:1-60 (2000).

One role of surfactants in a formulation can be to prevent or minimize aggregation and/or adsorption such as surface-induced degradation. At sufficient concentrations, generally about the surfactant's micellar concentration, a surface layer of surfactant molecules can serve to prevent protein molecules from adsorbing at the interface. Thereby, surface-induced degradation can be minimized. Surfactants, their use, formulation and characteristics for formulations are known in the art and can be found described in, for example, Randolph T.W. and Jones L.S., Surfactant-protein interactions. Pharm. Biotechnol. 13:159-75 (2002).

A surfactant for inclusion in a formulation can be chosen, for example, to enhance or promote retention in stability of the biopharmaceutical by preventing or reducing aggregation and/or adsorption. Sorbitan fatty acid esters such as the polysorbates are surfactants exhibiting a wide range of hydrophilic and emulsifying characteristics. They can be used individually or in combination with other surfactants to cover a wide range of stabilization needs. Such characteristics can be suitable for use with biopharmaceuticals because they can be tailored to cover the wide range of hydrophobic and hydrophilic characteristics of biopharmaceuticals. Considerations for selecting a surfactant include those described previously with reference to excipients in general as well as the hydrophobic character and critical micellar concentration of the surfactant. The surfactants exemplified herein, as well as many others well known in the art can be used in formulations described in the specification.

Surfactant concentration ranges for a formulation include those described previously with reference to excipients in general, for example, useful concentrations can be less than about 1% (w/v). In this regard, surfactant concentrations generally can be used at ranges between about 0.001-0.10 % (w/v), between about 0.002-0.05% (w/v), between about 0.003-0.01% (w/v), between about 0.004-0.008% (w/v) or between about 0.005-0.006% (w/v). Surfactant concentrations and/or amounts less than, greater than or in between these ranges also can also be used. For example, one or more surfactants can be included in a formulation which constitute less than about 0.001% (w/v). Similarly, a formulation can contain a concentration of one or more surfactants greater than about 0.10% (w/v). Accordingly, a formulation can be produced that contains essentially any desired concentration or amount of one or more surfactants including, for example, about 0.001, 0.002, 0.003, 0.004, 0.005, 0.006, 0.007, 0.008, 0.009, 0.010, 0.02, 0.03, 0.04, 0.05, 0.06, 0.07, 0.08, 0.09 or 0.10% (w/v) or more.

Surfactants useful in either a liquid or lyophilized formulation can include, for example, sugar esters such as esters lauric acid (C12), palmitic acid (C16), stearic acid (C18), macrogol cetostearyl ethers, macrogol lauryl ethers, macrogol oleyl ether, macrogol oleate, macrogol stearate, macrogol glycerol ricinoleate, macrogol glycerol hydroxystearate; alkyl polyglucosides such as octyl glucoside and decyl maltoside; fatty alcohols such as cetyl alcohol and oleyl alcohol, and cocamides such as cocamide MEA, DEA, TEA, other non-ionic surfactants and other ionic surfactants.

The formulations provided herein can comprise a therapeutic polypeptide as a component of the formulation. The therapeutic polypeptide is an antibody or an antigen-binding fragment.

Given the teachings and guidance provided herein, those skilled in the art will understand that a formulation described herein can be equally applicable to many types of biopharmaceuticals, including those exemplified, as well as others known in the art. Given the teachings and guidance provided herein, those skilled in the art also will understand that the selection of, for example, type(s) or and/or amount(s) of one or more excipients, surfactants and/or optional components can be made based on the chemical and functional compatibility with the biopharmaceutical to be formulated and/or the mode of administration as well as other chemical, functional, physiological and/or medical factors well known in the art. For example, as described previously, non-reducing sugars exhibit favorable excipient properties when used with polypeptide biopharmaceuticals compared to reducing sugars. Accordingly, the formulations of the invention are exemplified further below with reference to polypeptide biopharmaceuticals.

In various embodiments, various types of polypeptide biopharmaceuticals applicable for use in a formulation can include different types of therapeutic polypeptides, for example, the immunoglobulin superfamily of polypeptides, growth factors, cytokines, cell signaling molecules and hormones. Exemplary polypeptide biopharmaceuticals applicable for use in a formulation can include many different therapeutic polypeptides including, for example, antibodies and functional fragments thereof, interleukins, G-CSF, GM-CSF, kinases, TNF and TNFR ligands including Fhm, cyclins, erythropoietin, nerve growth factor (NGF), developmentally regulated nerve growth factor-induced gene VGF, neurotrophic factors, neurotrophic factor NNT-1, Eph receptor, Eph receptor ligands; Eph-like receptor, Eph-like receptor ligands, inhibitors of apoptosis proteins (IAP), Thy-1 specific protein, Hek ligand (hek-L), Elk receptor and Elk receptor ligands, STATs, collagenase inhibitor, osteoprotegerin (OPG), APRIL/G70, AGP-3/BLYS, BCMA, TACI, Her-2/neu, Apolipoprotein polypeptides, integrins, extendins, insulins, growth hormones, follicle stimulating hormones, gonatdotropins, tissue inhibitor of metalloproteinases, C3b/C4b complement receptor, SHC binding protein, DKR polypeptides, extracellular matrix polypeptides, antibodies to the above therapeutic polypeptides and antibody functional fragments thereof, antibodies to receptors for the above therapeutic polypeptides and antibody functional fragments thereof, functional polypeptide fragments thereof, fusion polypeptides, and chimeric polypeptides.

Examples of commercially available biopharmaceuticals that can be used in various embodiments of the formulations can include, for example, (Etanercept; a CHO expressed dimeric fusion protein (Amgen Inc.)); (Epoetin alfa; a mammalian cell expressed glycoprotein (Amgen Inc.)); (Interferon alfacon-1; an *E. coli* expressed recombinant protein (Amgen Inc.)); (anakinra; an *E. coli* expressed recombinant, nonglycosylated form of the human interleukin-1 receptor antagonist (IL-1Ra) (Amgen Inc.)); (darbepoetin alfa; a CHO expressed recombinant human erythropoiesis stimulating protein (Amgen Inc.)); (pegfilgrastim; covalent conjugate of recombinant methionyl human G-CSF and 20kD PEG (Amgen Inc.)); (Filgrastim; an *E. coli* expressed human granulocyte colony-stimulating factor (G-CSF) (Amgen Inc.)), (Ancestim, stem cell factor; an E.Coli expressed recombinant human protein (Amgen Inc.)), (panitumumab; an antibody to EGF receptor (Amgen Inc.)) or denosumab (an antibody to RANKL (Amgen Inc.)). These and other available biopharmaceuticals can be used in formulations described herein, at the time of production, prior to use and/or prior to short or long term storage.

A biopharmaceutical can be an antibody. Described below are antibodies and functional fragments thereof and antigen-binding fragments, that can be employed as therapeutic polypeptides in various embodiments. As described previously, the chemical and physical properties, formulation considerations and methodology applicable to antibodies and functional fragments thereof, can be similarly applicable to biopharmaceuticals including polypeptide biopharmaceuticals.

An antibody or immunoglobulin is a polypeptide that has specific affinity for a molecular target or antigen. The target may be naturally occurring in any species, including human, cynomolgus monkeys, mice, dogs, cats and rabbits. In various embodiments, the target can be a variant of a naturally occurring protein. Such variants include variants with one or more amino acid substitutions, deletions, or additions. In certain embodiments, the target includes deletions of one or more domains of a naturally occurring protein.

Antibodies can be monoclonal or polyclonal. A monoclonal antibody can refer to an antibody that is the product of a single cell clone or hybridoma. Monoclonal antibody can also refer to an antibody produced by recombinant methods from heavy and light chain encoding immunoglobulin genes to produce a single molecular immunoglobulin species. Amino acid sequences for antibodies within a monoclonal antibody preparation are substantially homogeneous and the binding activity of antibodies within such a preparation can exhibit substantially the same antigen-binding activity when compared in the same or similar binding assay. As described further below, various antibody and monoclonal antibody characteristics are known in the art.

Monoclonal antibodies can be prepared using a wide variety of methods known in the art including the use of hybridoma, recombinant, myeloma cell-line expressed, phage display and combinatorial antibody library methodologies, or a combination thereof. For example, monoclonal antibodies can be produced using hybridoma techniques including those known in the art and taught, for example, in Harlow and Lane., Antibodies: A Laboratory Manual, Cold Spring Harbor Laboratory Press (1989); Hammerling, et al., in: Monoclonal Antibodies and T-Cell Hybridomas 563-681, Elsevier, N.Y. (1981); Harlow et al., Using Antibodies: A Laboratory Manual, Cold Spring Harbor Laboratory Press (1999), and Antibody Engineering: A Practical Guide, C.A.K. Borrebaeck, Ed., W.H. Freeman and Co., Publishers, New York, pp. 103-120 (1991). Examples of known methods for producing monoclonal antibodies by recombinant, phage display and combinatorial antibody library methods, including libraries derived from immunized and naive animals can be found described in Antibody Engineering: A Practical Guide, C.A.K. Borrebaeck, Ed., W.H. Freeman and Co., Publishers, New York, pp. 103-120 (1991).

A monoclonal antibody for use as a biopharmaceutical is not limited to antibodies produced through hybridoma technology. Rather, as described previously, a monoclonal antibody refers to an antibody that is derived from a single clone, including any eukaryotic, prokaryotic, or phage clone, and not necessarily by the method which it is produced.

An antibody functional fragment or antigen-binding fragment refers to a portion of an antibody which retains some or all of its target-specific binding activity. Such functional fragments can include, for example, fragments such as Fd, Fv, Fab, F(ab'), F(ab)2, F(ab')2, single chain Fv (scFv), chimeric antibodies, diabodies, triabodies, tetrabodies, peptibody, and minibody. Other functional fragments can include, for example, heavy (H) or light (L) chain polypeptides, variable heavy (VH) and variable light (VL) chain region polypeptides, complementarity determining region (CDR) polypeptides, single domain antibodies, and polypeptides that contain at least a portion of an immunoglobulin that is sufficient to retain target-specific binding activity.

"Peptibody" refers to a molecule comprising an antibody Fc domain (i.e., C_{H}2 and C_{H}3 antibody domains) that excludes antibody C_{H}1, C_{L}, V_{H}, and V_{L} domains as well as Fab and F(ab)2, wherein the Fc domain is attached to one or more peptides, preferably a pharmacologically active peptide, particularly preferably a randomly generated pharmacologically active peptide. The production of peptibodies is generally described in PCT publication WO00/24782, published May 4, 2000.

Peptibodies, are also included herein as an antibody functional fragment. Such antibody binding fragments can be found described in, for example, Harlow and Lane, supra; Molec. Biology and Biotechnology: A Comprehensive Desk Reference (Myers, R.A. (ed.), New York: VCH Publisher, Inc.); Huston et al., Cell Biophysics, 22:189-224 (1993); Plückthun and Skerra, Meth. Enzymol., 178:497-515 (1989) and in Day, E.D., Advanced Immunochemistry, Second Ed., Wiley-Liss, Inc., New York, NY (1990).

With respect to antibodies and functional antibody fragments thereof that exhibit beneficial binding characteristics to a target molecule, various forms, alterations and modifications are known in the art. Target-specific monoclonal antibodies for use in a formulation can include any of such various monoclonal antibody forms, alterations and modifications. Examples of such various forms and terms as they are known in the art are set forth below.

A Fab fragment refers to a monovalent fragment consisting of the VL, VH, CL and CH1 domains; a F(ab')2 fragment is a bivalent fragment comprising two Fab fragments linked by a disulfide bridge at the hinge region but lacking the Fc; a Fd fragment consists of the VH and CH1 domains; an Fv fragment consists of the VL and VH domains of a single arm of an antibody; and a dAb fragment (Ward et al., Nature 341:544-546, (1989)) consists of a VH domain.

An antibody can have one or more binding sites. If there is more than one binding site, the binding sites may be identical to one another or may be different. For example, a naturally occurring immunoglobulin has two identical binding sites, a single-chain antibody or Fab fragment has one binding site, while a "bispecific" or "bifunctional" antibody has two different binding sites.

A single-chain antibody (scFv) refers to an antibody in which a VL and a VH region are joined via a linker (e.g., a synthetic sequence of amino acid residues) to form a continuous polypeptide chain wherein the linker is long enough to allow the protein chain to fold back on itself and form a monovalent antigen-binding site (see, e.g., Bird et al., Science 242:423-26 (1988) and Huston et al., Proc. Natl. Acad. Sci. USA 85:5879-83 (1988)). Diabodies refer to bivalent antibodies comprising two polypeptide chains, wherein each polypeptide chain comprises VH and VL domains joined by a linker that is too short to allow for pairing between two domains on the same chain, thus allowing each domain to pair with a complementary domain on another polypeptide chain (see, e.g., Holliger et al., Proc. Natl. Acad. Sci. USA 90:6444-48 (1993), and Poljak et al., Structure 2:1121-23 (1994)). If the two polypeptide chains of a diabody are identical, then a diabody resulting from their pairing will have two identical antigen-binding sites. Polypeptide chains having different sequences can be used to make a diabody with two different antigen-binding sites. Similarly, tribodies and tetrabodies are antibodies comprising three and four polypeptide chains, respectively, and forming three and four antigen-binding sites, respectively, which can be the same or different.

A CDR refers to a region containing one of three hypervariable loops (H1, H2 or H3) within the non-framework region of the immunoglobulin (Ig or antibody) VH β-sheet framework, or a region containing one of three hypervariable loops (L1, L2 or L3) within the non-framework region of the antibody VL β-sheet framework. Accordingly, CDRs are variable region sequences interspersed within the framework region sequences. CDR regions are known to those skilled in the art and have been defined by, for example, Kabat as the regions of most hypervariability within the antibody variable (V) domains (Kabat et al., J. Biol. Chem. 252:6609-6616 (1977); Kabat, Adv. Prot. Chem. 32:1-75 (1978)). CDR region sequences also have been defined structurally by Chothia as those residues that are not part of the conserved β-sheet framework, and thus are able to adapt different conformations (Chothia and Lesk, J. Mol. Biol. 196:901-917 (1987)). Both terminologies are well recognized in the art. The positions of CDRs within a canonical antibody variable domain have been determined by comparison of numerous structures (Al-Lazikani et al., J. Mol. Biol. 273:927-948 (1997); Morea et al., Methods 20:267-279 (2000)). Because the number of residues within a loop varies in different antibodies, additional loop residues relative to the canonical positions are conventionally numbered with a, b, c and so forth next to the residue number in the canonical variable domain numbering scheme (Al-Lazikani et al., supra (1997)). Such nomenclature is similarly known to those skilled in the art.

For example, CDRs defined according to either the Kabat (hypervariable) or Chothia (structural) designations, are set forth in the table below.

**Table 1: CDR Definitions**

| | Kabat¹ | Chothia² | Loop Location |
|---|---|---|---|
| V_{H} CDR1 | 31-35 | 26-32 | linking B and C strands |
| V_{H} CDR2 | 50-65 | 53-55 | linking C' and C" strands |
| V_{H} CDR3 | 95-102 | 96-101 | linking F and G strands |
| V_{L} CDR1 | 24-34 | 26-32 | linking B and C strands |
| V_{L} CDR2 | 50-56 | 50-52 | linking C' and C" strands |
| V_{L} CDR3 | 89-97 | 91-96 | linking F and G strands |

| | | | |
|---|---|---|---|
| ¹ Residue numbering follows the nomenclature of Kabat et al., *supra* ² Residue numbering follows the nomenclature of Chothia et al., *supra* | | | |

A chimeric antibody refers to an antibody that contains one or more regions from one antibody and one or more regions from one or more other antibodies. In one example, one or more of the CDRs are derived from a non-human donor antibody having specific activity to a target molecule and the variable region framework is derived from a human recipient antibody. In another example, all of the CDRs can be derived from a non-human donor antibody having specific activity to a target molecule and the variable region framework is derived from a human recipient antibody. In yet another specific example, the CDRs from more than one non-human target-specific antibodies are mixed and matched in a chimeric antibody. For instance, a chimeric antibody can include a CDR1 from the light chain of a first non-human target-specific antibody, a CDR2 and a CDR3 from the light chain of a second non-human target-specific antibody and the CDRs from the heavy chain from a third target-specific antibody. Further, the framework regions can be derived from one of the same or from one or more different human antibodies or from a humanized antibody. Chimeric antibodies can be produced where both the donor and recipient antibodies are human.

A humanized antibody or grafted antibody has a sequence that differs from a non-human species antibody sequence by one or more amino acid substitutions, deletions, and/or additions, such that the humanized antibody is less likely to induce an immune response, and/or induces a less severe immune response, as compared to the non-human species antibody, when it is administered to a human subject. In one example, certain amino acids in the framework and constant domains of the heavy and/or light chains of the non-human species antibody can be changed to produce the humanized antibody. In another example, the constant domain(s) from a human antibody can be fused to the variable domain(s) of a non-human species. Examples of how to make humanized antibodies can be found in U.S. Pat. Nos. 6,054,297, 5,886,152 and 5,877,293. Humanized antibodies also include antibodies produced using antibody resurfacing methods.

A human antibody refers to antibodies that have one or more variable and constant regions derived from human immunoglobulin sequences. For example, a fully human antibody includes an antibody where all of the variable and constant domains are derived from human immunoglobulin sequences. Human antibodies can be prepared using a variety of methods known in the art.

One or more CDRs also can be incorporated into a molecule either covalently or noncovalently to make it an immunoadhesin. An immunoadhesin can incorporate the CDR(s) as part of a larger polypeptide chain, can covalently link the CDR(s) to another polypeptide chain, or can incorporate the CDR(s) noncovalently. The CDRs permit the immunoadhesin to specifically bind to a particular antigen of interest.

A neutralizing antibody or an inhibitory antibody refers to a target-specific monoclonal antibody that inhibits the binding of the target molecule to its binding partner when an excess of the target-specific monoclonal antibody reduces the amount of binding partner bound to the target. Binding inhibition can occur by at least about 10%, particularly by at least about 20%. In various specific examples, the monoclonal antibody can reduce the amount of binding partner bound to the target by, for example, at least about 30%, 40%, 50%, 60%, 70%, 75%, 80%, 85%, 90%, 95%, 97%, 99%, and 99.9%. The binding reduction can be measured by any means known to one of ordinary skill in the art, for example, as measured in an in vitro competitive binding assay.

An antagonistic antibody refers to an antibody that inhibits the activity of a target molecule when added to a cell, tissue or organism expressing the target molecule. Diminution in activity can be by at least about 5%, particularly by at least about 10%, more particularly, by at least about 15% or more, compared to the level of target molecule activity in the presence of binding partner alone. In various specific examples, the target-specific monoclonal antibodies for use as a biopharmaceutical of the invention can inhibit the target molecule activity by at least about 20%, 30%, 40%, 50%, 60%, 70%, 80%, 90% or 100%.

As with the above described target-specific monoclonal antibodies, in further embodiments, target-specific monoclonal antibodies for use in various embodiments can include monoclonal antibodies that exhibit target molecule antagonistic activity. An antagonist of target molecule activity decreases at least one function or activity of the target molecule when bound or stimulated by its binding partner. Such functions can include, for example, stimulation or inhibition of cell regulation, gene regulation, protein regulation, signal transduction, cell proliferation, differentiation, migration, cell survival or any other biochemical and/or physiological function. Other functions or activities of a target molecule also can be reduced or inhibited by antagonistic target-specific monoclonal antibodies for use as a biopharmaceutical of the invention. Given the teachings and guidance provided herein, those skilled in the art will be able to make and identify a wide range of target-specific monoclonal antibodies exhibiting different antagonistic activities.

Antagonistic target-specific monoclonal antibodies can be produced and identified as described herein. A specific method for identifying antagonistic target-specific monoclonal antibodies includes contacting a target-specific monoclonal antibody with a target molecule expressing cell that is responsive to its binding partner in the presence of binding partner or other agonist. Contacting is performed under conditions sufficient for binding and a decrease or reduction in a target molecule function or activity can be determined. Those target-specific monoclonal antibodies that decrease, reduce or prohibit at least one function or activity of the target are identified as being a target-specific antagonistic monoclonal antibody.

An agonist antibody refers to an antibody that activates a target molecule by at least about 5%, particularly by at least about 10%, or about 15% when added to a cell, tissue or organism expressing the target molecule, where 100% activation is the level of activation achieved under physiological conditions by the same molar amount of binding partner. In various specific examples, the target-specific monoclonal antibodies for use as a biopharmaceutical of the invention can activate target molecule activity by at least about 20%, 30%, 40%, 50%, 60%, 70%, 80%, 90%, 100%, 125%, 150%, 175%, 200%, 250%, 300%, 350%, 400%, 450%, 500%, 750% or 1000%.

In further embodiments, target-specific monoclonal antibodies for use in various embodiments can include monoclonal antibodies that exhibit target molecule agonistic activity. An agonistic of target molecule activity refers to a molecule that increases at least one function or activity of the target molecule when bound to its binding partner. Activities that can be increased include, for example, those described previously with respect to antagonistic activities. Accordingly, target-specific monoclonal antibodies having target molecule antagonist activity decrease, reduce or prevent one or more cellular functions or activities of a target molecule. Target-specific monoclonal antibodies having target molecule agonist activity increase, promote or stimulate one or more cellular functions or activities of a target molecule. Given the teachings and guidance provided herein, those skilled in the art will be able to make and identify a wide range of target-specific monoclonal antibodies exhibiting different antagonistic or agonistic activities.

Given the teachings and guidance provided herein, those skilled in the art can employ immunization methods, hybridoma production, myeloma cell-line expression and screening methods well known in the art to produce agonistic target-specific monoclonal antibodies. A method for identifying agonistic target-specific monoclonal antibodies includes contacting a target-specific monoclonal antibody with a target molecule expressing cell that is responsive to the target molecule binding partner under conditions sufficient for binding and determining stimulation or increase in a target molecule function or activity. Those target-specific monoclonal antibodies that increase, stimulate or promote at least one function or activity of target molecule are identified as being a target-specific agonistic monoclonal antibody.

An epitope refers to a part of a molecule, for example, a portion of a polypeptide, that specifically binds to one or more antibodies within the antigen-binding site of the antibody. Epitopic determinants can include continuous or non-continuous regions of the molecule that bind to an antibody. Epitopic determinants also can include chemically active surface groupings of molecules such as amino acids or sugar side chains and have specific three dimensional structural characteristics and/or specific charge characteristics.

Specific binding refers to a target-specific monoclonal antibody exhibiting preferential binding for a target molecule compared to other related but non-target molecules or compared to other non-target molecules. Preferential binding includes a monoclonal antibody for use as a biopharmaceutical of the invention exhibiting detectable binding to its target molecule while exhibiting little or no detectable binding to another related but non-target molecule.

Specific binding can be determined by any of a variety of measurements known to those skilled in the art including, for example, affinity (Kₐ or K_{d}), association rate (kₒₙ), dissociation rate (k_{off}), avidity or a combination thereof. Any of a variety of methods or measurements well known in the art can be employed and are applicable for determining target-specific binding activity. Such methods and measurements include, for example, apparent or relative binding between a target molecule and a non-target molecule. Both quantitative and qualitative measurements can be employed for making such apparent or relative binding determinations. Specific examples of binding determinations include, for example, competitive binding assays, protein or Western blot methodology, ELISA, RIA, surface plasmon resonance, evanescent wave methodology, flow cytometry and/or confocal microscopy.

Further, specific binding of antagonistic or agonistic target-specific monoclonal antibodies can be determined by any of the methods described above or below including, for example, determining a change in a cellular function or activity. Methods for measuring a change in cellular function or activity such as proliferation, differentiation or other biochemical and/or physiological function are known in the art. As with the binding assays described previously, both quantitative and qualitative measurements can be employed for making apparent or relative determinations with respect to antagonizing or agonizing one or more cellular functions.

Target-specific monoclonal antibodies for use as a biopharmaceutical of the invention, or functional fragments thereof, can be produced in any of the various antibody forms and/or can be altered or modified in any of the various ways as described previously while still maintaining their specific target binding activity. Any of such antibody forms, alterations or modifications, including combinations thereof, of a target-specific monoclonal antibody, or functional fragment thereof, can be used as a biopharmaceutical. Any of such various antibody forms, alterations or modifications of a target-specific monoclonal antibody for use as a biopharmaceutical, or a functional fragment thereof, can similarly be used in the methods, compositions and/or articles of manufacture described herein. For example, target-specific monoclonal antibodies, or functional fragments thereof, can include target-specific grafted, humanized, Fd, Fv, Fab, F(ab)2, scFv and peptibody monoclonal antibodies as well as all other forms, alterations and/or modifications described previously, and including other forms well known to those skilled in the art.

Methods for producing hybridomas and screening for target-specific monoclonal antibodies using hybridoma technology are known in the art. For example, mice can be immunized with a target molecule such as a polypeptide and once an immune response is detected, e.g., antibodies specific for the target molecule are detected in the mouse serum, the mouse spleen is harvested and splenocytes isolated. The splenocytes are then fused by well known methods to any suitable myeloma cells, for example, cells from cell line SP20 available from the ATCC. Hybridomas are selected and cloned by limited dilution. The hybridoma clones are then assayed by methods known in the art for cells that secrete antibodies capable of binding a target molecule. Ascites fluid, which generally contains high levels of antibodies, can be generated by immunizing mice with positive hybridoma clones.

Additionally, recombinant expression in prokaryotic or eukaryotic hosts can be used to generate target-specific monoclonal antibodies. Recombinant expression can be utilized to produce single target-specific monoclonal antibody species, or functional fragments thereof. Alternatively, recombinant expression can be utilized to produce diverse libraries of heavy and light, or variable heavy and variable light chain combinations, and then screened for a monoclonal antibody, or functional fragment thereof, exhibiting specific binding activity to the target molecule. For example, heavy and light chains, variable heavy and light chain domains, or functional fragments thereof, can be co-expressed from nucleic acids encoding target-specific monoclonal antibodies using methods well known in the art to produce specific monoclonal antibody species. Libraries can be produced using methods well known in art from co-expressed populations of nucleic acids encoding heavy and light chains, variable heavy and light chain domains, or functional fragments thereof, and screened by affinity binding to the target molecule for identification of target-specific monoclonal antibodies. Such methods can be found described in, for example, Antibody Engineering: A Practical Guide, C.A.K. Borrebaeck, Ed., supra; Huse et al., Science 246:1275-81 (1989); Barbas et al., Proc. Natl. Acad. Sci. USA 88:7978-82 (1991); Kang et al., Proc. Natl. Acad. Sci. USA 88:4363-66 (1991); Plückthun and Skerra, supra; Felici et al., J. Mol. Biol. 222:301-310 (1991); Lerner et al., Science 258:1313-14 (1992), and in U.S. Patent No. 5,427,908.

Cloning of encoding nucleic acids can be accomplished using methods well known to those skilled in the art. Similarly, cloning of heavy and/or light chain repertoires of encoding nucleic acid, including VH and/or VL encoding nucleic acids also can be accomplished by methods well known to those skilled in the art. Such methods include, for example, expression cloning, hybridization screening with a complementary probe, polymerase chain reaction (PCR) using a complementary pair of primers or ligase chain reaction (LCR) using a complementary primer, and reverse transcriptase PCR (RT-PCR). Such methods can be found described in, for example, Sambrook et al., Molecular Cloning: A Laboratory Manual, Third Ed., Cold Spring Harbor Laboratory, New York (2001) and Ansubel et al., Current Protocols in Molecular Biology, John Wiley and Sons, Baltimore, MD (1999).

Encoding nucleic acids also can be obtained from any of various public databases including whole genome databases such as those operated by The National Center for Biotechnology Information (NCBI) of the National Institutes of Health (NIH). A particularly useful method of isolating either a single encoding nucleic or a repertoire of encoding nucleic acids for heavy and/or light chains, or functional fragments thereof, can be accomplished without specific knowledge of the coding region portion because primers are available or can be readily designed using conserved portions of antibody variable or constant region portions. For example, a repertoire of encoding nucleic acids can be cloned using a plurality of degenerate primers to such regions together with PCR. Such methods are known in the art and can be found described in, for example, Huse et al., supra, and Antibody Engineering: A Practical Guide, C.A.K. Borrebaeck, Ed., supra. Any of the above methods as well as others known in the art, including combinations thereof, can be used to generate a target-specific monoclonal antibody for use as a biopharmaceutical of the invention.

In various embodiments, a formulation is provided having an antibody or a functional fragment of an antibody as a therapeutic polypeptide. The therapeutic polypeptide can include a monoclonal antibody, Fd, Fv, Fab, F(ab'), F(ab)2, F(ab')2, single chain Fv (scFv), chimeric antibodies, diabodies, triabodies, tetrabodies, minibodies or peptibodies.

Concentrations of the antibody or functional fragment of the antibody can vary, for example, depending on the activity of the biopharmaceutical, the indication to be treated, mode of administration, the treatment regime and whether the formulation is intended for long term storage in either liquid or lyophilized form. Those skilled in the art can determine without undue experimentation approximate biopharmaceutical concentrations. There are more than 80 biopharmaceuticals approved for therapeutic use in the United States for a wide range of medical indications, modes of administration and treatment regimes. These approved biopharmaceuticals, as well as others, can be exemplary of the range of biopharmaceutical concentrations that can be used in various embodiments.

Generally, a biopharmaceutical, for example, a therapeutic polypeptide biopharmaceutical, can be included in the formulation of various embodiments at a concentration from between about 1-200 mg/ml, about 10-200 mg/ml, about 20-180 mg/ml, between about 30-160 mg/ml, between about 40-120 mg/ml, or between about 50-100 mg/ml, about 60-80 mg/ml. or about 30-50 mg/ml.

In various embodiments, the biopharmaceutical can be an antibody or antigen-binding fragment having a concentration from between about 3 to about 70 mg/ml, about 5 to about 60mgl/ml, about 10 to about 50mg/ml, about 20 to about 40 mg/ml, about 30 to about 100 mg/ml, or about 40 to about 200 mg/ml.

Biopharmaceutical concentrations and/or amounts less than, greater than or in between these ranges also can be used in formulations described herein. For example, one or more biopharmaceuticals can be included in a formulation at less than about 1.0 mg/ml. Similarly, a formulation can contain a concentration of one or more biopharmaceuticals greater than about 200 mg/ml, particularly when formulated for storage. Accordingly, a formulation of the invention can be produced that contains a desired concentration or amount of one or more biopharmaceuticals including, for example, about 1, 5, 10, 15, 20, 21, 22, 23, 24, 25, 26, 27, 28, 29, 30, 31, 32, 35, 36, 37, 38, 39, 40, 45, 50, 55, 60, 65, 70, 75, 80, 85, 90, 95, 100, 110, 120, 130, 140, 150, 160, 170, 180, 190 or 200 mg./ml or more. In examples below, results are provided for a formulation having a therapeutic polypeptide (an antibody) at a concentration of about 3 mg/ml, about 30 mg/ml., about 40 mg/ml or about 100 mg/ml.

In various embodiments, a formulation can include combinations of biopharmaceuticals in the formulation. For example, a formulation of the invention can include a single biopharmaceutical for treatment of one or more conditions. A formulation of the invention also can include two or more different biopharmaceuticals for a single or multiple conditions. Use of multiple biopharmaceuticals in a formulation of the invention can be directed to, for example, the same or different indications. Similarly, multiple biopharmaceuticals can be used in a formulation of the invention to treat, for example, both a pathological condition and one or more side effects caused by the primary treatment. Multiple biopharmaceuticals also can be included in a formulation of the invention to accomplish different medical purposes including, for example, simultaneous treatment and monitoring of the progression of the pathological condition. Multiple, concurrent therapies such as those exemplified above as well as other combinations well known in the art are particularly useful for patient compliance because a single formulation can be sufficient for some or all suggested treatments and/or diagnosis. Those skilled in the art will know those biopharmaceuticals that can be admixed for a wide range of combination therapies. Similarly, in various embodiments, a formulation can be used with a small molecule drug and combinations of one or more biopharmaceuticals together with one or more small molecule pharmaceuticals. Therefore, in various embodiments a formulation is provided containing 1, 2, 3, 4, 5 or 6 or more different biopharmaceuticals, as well as, for one or more biopharmaceuticals combined with one or more small molecule pharmaceuticals.

In various embodiments, a formulation can include one or more preservatives and/or additives known in the art. Similarly, a formulation can further be formulated into any of various known delivery formulations. For example, a formulation can include lubricating agents, emulsifying agents, suspending agents, preserving agents such as methyl- and propylhydroxybenzoates, sweetening agents and flavoring agents. Such optional components, their chemical and functional characteristics are known in the art. Similarly known in the art are formulations that facilitate rapid, sustained or delayed release of the biopharmaceutical after administration. A formulation of the invention can be produced to include these or other formulation components known in the art.

Once a formulation is prepared as described herein, stability of the one or more biopharmaceuticals contained within the formulation can be assessed using methods known in the art. Several methods are exemplified below in the Examples and include size exclusion chromatography, particle counting and cation exchange chromatography. Other methods can comprise any of a variety of functional assays including, for example, binding activity, other biochemical activity and/or physiological activity can be assessed at two or more different time points to determine the stability of the biopharmaceutical in the buffered formulation of the invention.

A formulation can, in general, be prepared according to pharmaceutical standards and using pharmaceutical grade reagents. Similarly, a formulation can be prepared using sterile reagents in a sterile manufacturing environment or sterilized following preparation. Sterile injectable solutions can be prepared using known procedures in the art including, for example, by incorporating one or more biopharmaceuticals in the required amount in a glutamic acid buffer (reference example) or excipient with one or a combination of formulation components described herein followed by sterilization micro filtration. In various embodiments, sterile powders for the preparation of sterile injectable solutions can include, for example, vacuum drying and freeze-drying (lyophilization). Such drying methods will yield a powder of the one or more biopharmaceuticals together with any additional desired components from a previously sterile-filtered solution thereof.

Administration and dosage regimens can be adjusted to provide an effective amount for an optimum therapeutic response. For example, a single bolus can be administered, several divided doses can be administered over time or the dose can be proportionally reduced or increased as indicated by the exigencies of the therapeutic situation. It can be useful to formulate a formulation for intravenous, parenteral or subcutaneous injection in a unit dosage form for ease of administration and uniformity of dosage in administering an effective amount of one or more biopharmaceuticals. Unit dosing refers to a physically discrete amount of pharmaceutical suited as unitary dosages for the subjects to be treated; each unit contains a predetermined quantity of active biopharmaceutical calculated to produce a desired therapeutic effect.

Dosing will depend on the antibody used in the formulation. If, for example, an anti-NGF antibody is used in the formulation described herein, dosing frequency will depend upon the pharmacokinetic parameters of that anti-NGF antibody used in the formulation. Typically, a clinician can administer the composition until a dosage is reached that achieves the desired effect. An example of a desired effect can be diminution of pain or neuropathic pain following administration of a formulation comprising anti-NGF antibodies.

The composition may therefore be administered as a single dose, or as two or more doses (which may or may not contain the same amount of the desired molecule) over time, or as a continuous infusion via an implantation device or catheter. Further refinement of the appropriate dosage is routinely made by those of ordinary skill in the art and is within the ambit of tasks routinely performed by them. Appropriate dosages may be ascertained through use of appropriate dose-response data. In various embodiments, the antibodies in formulations described herein can be administered to patients throughout an extended time period. Chronic administration of a fully human antibody can minimize the adverse immune or allergic response that can be associated with antibodies that are raised against a human antigen in a non-human animal, for example, a non-fully human antibody produced in a non-human species.

The effective amount of an anti-NGF antibody-containing pharmaceutical or any other antibody-containing formulation to be employed therapeutically can depend, for example, upon the therapeutic context and objectives. One skilled in the art will appreciate that the appropriate dosage levels for treatment will vary depending, in part, upon the molecule delivered, the indication for which the anti-NGF antibody is being used, the route of administration, and the size (body weight, body surface or organ size) and/or condition (the age and general health) of the patient. In certain embodiments, the clinician may titer the dosage and modify the route of administration to obtain the optimal therapeutic effect. A typical dosage can range from about 0.1 µg/kg to up to about 30 mg/kg or more, depending on the factors mentioned above. In preferred embodiments, the dosage may range from about 0.1 µg/kg up to about 30 mg/kg; more preferably from about 1 µg/kg up to about 30 mg/kg; or even more preferably from about 5 µg/kg up to about 30 mg/kg. It can also be envisaged that under appropriate conditions as recognized by one of skill in the art, dosages higher than 30 mg/kg can be administered, provided that the benefits of such a dosing are not outweighed by any negative effects of administration of the larger doses.

In various embodiments, an effective amount of a polypeptide biopharmaceutical such as a therapeutic antibody, or functional fragment thereof, can be administered, for example, more than once, at scheduled intervals over a period of time. The therapeutic antibody can be administered over a period of at least a month or more including, for example, one, two, or three months or longer. For treating chronic conditions, long-term, sustained treatment is generally most effective. Shorter periods of administration can be sufficient when treating acute conditions including periods, for example, from one to six weeks. In general, a therapeutic antibody or other biopharmaceutical is administered until the patient manifests a medically relevant degree of improvement over baseline for the chosen indicator or indicators.

Depending on the selected biopharmaceutical and indication to be treated, a therapeutically effective amount is sufficient to cause a reduction in at least one symptom of the targeted pathological condition by at least about 1%, 5%, 10%, 15%, 20%, 25%, 30%, 35%, 40%, 45%, 50%, 55% or 60% or more, relative to untreated subjects. The ability of a formulation to reduce or inhibit a symptom can be evaluated, for example, in an animal model system predictive of efficacy for the targeted condition in human. Alternatively, the ability of a formulation to reduce or inhibit a symptom can be evaluated, for example, by examining an *in vitro* function or activity of the formulation indicative of in vivo therapeutic activity.

Actual dosage levels of one or more biopharmaceuticals in a formulation can be varied so as to obtain an amount of the active biopharmaceutical which is effective to achieve the desired therapeutic response for a particular patient, formulation, and mode of administration, without being toxic to the patient. One skilled in the art would be able to determine administered amounts based on factors such as the subject's size, the severity of the subject's symptoms, and the selected biopharmaceutical and/or route of administration. The selected dosage level can depend, for example, upon a variety of pharmacokinetic factors including the activity of the biopharmaceutical employed, the route of administration, the time of administration, the rate of excretion, the duration of the treatment, other drugs, compounds and/or materials used in combination with the particular compositions employed, the age, sex, weight, condition, general health and prior medical history of the patient being treated, and like factors well known in the medical arts. Various embodiments can involve administering a therapeutic polypeptide such as an antibody, or functional fragment thereof, in a formulation of the invention at a dosage of from about 1 ng of antibody per kg of subject's weight per day (1 ng/kg/day) to about 10 mg/kg/day, more particularly from about 500 ng/kg/day to about 5 mg/kg/day, and even more particularly from about 5 µg/kg/day to about 2 mg/kg/day, to a subject. Higher doses can also be administered under appropriate conditions.

A physician or veterinarian having skill in the art can readily determine and prescribe the effective amount of the required pharmaceutical formulation. For example, the physician or veterinarian can initiate doses of a formulation of the invention at levels lower than that required in order to achieve the desired therapeutic effect and gradually increase the dosage until the desired effect is achieved. In general, a suitable daily dose of a formulation of the invention will be that amount of the biopharmaceutical which is the lowest dose effective to produce a therapeutic effect. Such an effective amount will generally depend upon the factors described previously. It is particularly useful that administration be intravenous, intramuscular, intraperitoneal, or subcutaneous. If desired, the effective daily dose to achieve an effective amount of a formulation can be administered as two, three, four, five, six or more sub-doses administered separately at appropriate intervals throughout the day, optionally, in unit dosing amounts.

In various embodiments, a formulation can be administered, for example, with medical devices known in the art. Medical devices for administration of the formulation can include syringes and autoinjectors. Syringes can be pre-filled syringes. In various embodiments, a formulation can be administered with a needleless hypodermic injection device, such as the devices described in U.S. Pat. Nos. 5,399,163; 5,383,851; 5,312,335; 5,064,413; 4,941,880; 4,790,824; or 4,596,556. Examples of known implants and modules that can be useful with formulations described herein include: U.S. Pat. No. 4,487,603, which describes an implantable micro-infusion pump for dispensing medication at a controlled rate; U.S. Pat. No. 4,486,194, which describes a therapeutic device for administering medicants through the skin; U.S. Pat. No. 4,447,233, which describes a medication infusion pump for delivering medication at a precise infusion rate; U.S. Pat. No. 4,447,224, which describes a variable flow implantable infusion apparatus for continuous drug delivery; U.S. Pat. No. 4,439,196, which describes an osmotic drug delivery system having multi-chamber compartments, and U.S. Pat. No. 4,475,196, which describes an osmotic drug delivery system. Many other such implants, delivery systems, and modules are known to those skilled in the art. Additionally, in various embodiments, the formulations can be administered from a pre-filled syringe.

In various embodiments, a biopharmaceutical for use in a formulation can be formulated to facilitate selective distribution *in vivo.* For example, the blood-brain barrier (BBB) excludes many highly hydrophilic compounds. To facilitate crossing of the BBB if desired, a formulation can additionally include, for example, liposomes for encapsulation of one or more biopharmaceuticals. For methods of manufacturing liposomes, see, for example, U.S. Pat. Nos. 4,522,811; 5,374,548; and 5,399,331. The liposomes can further contain one or more moieties which are selectively transported into specific cells or organs, thus enhancing targeted delivery of a selected biopharmaceutical (see, e.g., V. V. Ranade (1989) J. Clin. Pharmacol. 29:685). Exemplary targeting moieties include folate or biotin (see, e.g., U.S. Pat. No. 5,416,016 to Low et al.); mannosides (Umezawa et al., (1988) Biochem. Biophys. Res. Commun. 153:1038); antibodies (P. G. Bloeman et al. (1995) FEBS Lett. 357:140; M. Owais et al. (1995) Antimicrob. Agents Chemother. 39:180) or surfactant protein A receptor (Briscoe et al. (1995) Am. J. Physiol. 1233:134).

After preparation of the biopharmaceutical, for example, an antibody or antigen-binding fragment of interest, the pharmaceutical formulation comprising it can be prepared. Generally, the antibody or antigen-binding fragment to be formulated has not been subjected to lyophilization and is in a solution. The solution can be an aqueous solution. In various embodiments, however, prior lyophilization may have occurred. The therapeutically effective amount of antibody present in the formulation can be determined by taking into account the desired dose volumes and mode(s) of administration. For example, from about 0.1 mg/mL to about 60 mg/mL, from about 10g/mL to about 40g/mL or from about 20 mg/mL to about 35 mg/mL.

The present specification further discloses a method of preparing a formulation. The method includes combining an acetic acid buffer solution having a pH from about 4.0 to about 6.0, proline and an effective amount of a therapeutic polypeptide. One or more of the formulation components described herein can be combined with one or more effective amounts of a biopharmaceutical to produce a wide range of formulations.

An aqueous formulation can be prepared comprising the antibody in a pH-buffered solution. The buffer is an acetic acid buffer. The buffer can have a pH in the range from about 4.0 to about 6.0, from about 4.5 to about 5.5, or a pH of about 5.0. The buffer concentration can be from about 1 mM to about 50 mM, from about 5 mM to about 30 mM, about 10 mM or about 30 mM.

Briefly, with respect to compositions, kits and/or medicaments, the combined effective amounts of one or more biopharmaceuticals within a formulation can be included within a single container or more than one container.

According to various embodiments, the formulation can be essentially free of one or more preservatives, such as benzyl alcohol, phenol, m-cresol, chlorobutanol and benzethonium Cl. In other embodiments, however, a preservative can be included in the formulation, particularly where the formulation is a multidose formulation. The concentration of preservative can be in the range from about 0.1% to about 2% or from about 0.5% to about 1%.

One or more other pharmaceutically acceptable carriers, excipients or stabilizers such as those described in Remington's Pharmaceutical Sciences 16th edition, Osol, A. Ed. (1980) can be included in the formulation provided that they do not adversely affect the desired characteristics of the formulation.

The formulation described in this specification may also comprise more than one therapeutic protein as desired for the particular indication being treated, preferably those with complementary activities that do not adversely affect the other protein.

The formulations to be used for *in vivo* administration can be sterile. This can be accomplished by filtration through sterile filtration membranes, prior to, or following, preparation of the formulation.

Imaging components can optionally be included and the packaging also can include written or web-accessible instructions for using the formulation. A container can include, for example, a vial, bottle, syringe, pre-filled syringe or any of a variety of formats well known in the art for multi-dispenser packaging.

The antibody formulations described herein can be used to treat various conditions that require administration of therapeutic polypeptides. For example, if a condition in a patient is caused by increased expression of NGF or increased sensitivity to NGF, the formulation described herein can be used with an antibody or antigen-binding fragment to NGF. Such diseases have also been referred to as "NGF-mediated disease" or "NGF-mediated condition." Additional information, concerning conditions relating to NGF expression or sensitivity, "NGF-mediated disease" or "NGF-mediated condition," antibodies or antigen-binding fragment to NGF, can be found in, for example, US Patent Application Publication 2005/0074821A1. Antibody formulations to treat other conditions can also be prepared.

In various embodiments, an antibody or antigen-binding fragment in the formulation comprises an Fd, Fv, Fab, F(ab'), F(ab)₂, F(ab')₂, F(ab)₃, Fc, bis-scFv(s), single chain Fv (scFv), monoclonal antibodies, polyclonal antibodies, chimeric antibodies, diabodies, triabodies, tetrabodies, minibody, peptibody, VhH domain, V-NAR domain, V_{H} domain, V_{L} domain, camel Ig, Ig NAR, or receptibody. The antibody or antigen-binding fragment can bind a growth factor. The growth factor can be nerve growth factor. The formulation comprising NGF can have a concentration of NGF from about 10 to about 50mg/ml.

In various embodiments, the antibody or antigen-binding fragment in the formulation is at a concentration from between about 3 to about 70 mg/ml, about 5 to about 60mgl/ml, about 10 to about 50mg/ml, 20 to about 40 mg/ml, about 30 to about 100 mg/ml, or about 40 to about 200 mg/ml.

In various embodiments, the method comprises combining an antibody or antigen-binding fragment, wherein the antibody or an antigen-binding fragment comprises, an Fd, Fv, Fab, F(ab'), F(ab)₂, F(ab')₂, F(ab)₃, Fc, bis-scFv(s), single chain Fv (scFv), monoclonal antibodies, polyclonal antibodies, chimeric antibodies, diabodies, triabodies, tetrabodies, minibody, peptibody, VhH domain, V-NAR domain, V_{H} domain, V_{L} domain, camel Ig, Ig NAR, or receptibody.

In various embodiments, the method comprises combining a therapeutic polypeptide comprising a concentration from about 3 to about 70 mg/ml, about 5 to about 60mgl/ml, about 10 to about 50mg/ml, 20 to about 40 mg/ml, about 30 to about 100 mg/ml, or about 40 to about 200 mg/ml.

The present specification discloses kits comprising in one or more containers an antibody or antigen-binding formulation and proline. The kits can comprise in one more containers, acetic acid buffer, proline and an antibody or antigen-binding fragment and instructions regarding the use thereof. The kits can comprise a formulation that is a pharmaceutically acceptable formulation for human use. A kit can also comprise instructions for use thereof.

The kit can comprise a biopharmaceutical protein, wherein the protein is a biopharmaceutical protein formulated for the treatment of a disease in humans, for example an antibody or antigen-binding fragment. Kits can comprise one or more single or multi-chambered syringes (e.g., liquid syringes and lyosyringes) for administering one or more formulations described herein. An example of a lyosyringe is the Lyo-Ject™, a dual-chamber pre-filled lyosyringe available from Vetter GmbH, Ravensburg, Germany.

The kit can comprise formulation components for parenteral, subcutaneous, intramuscular or IV administration, sealed in a vial under partial vacuum in a form ready for loading into a syringe and administration to a subject. In this regard, the composition can be disposed therein under partial vacuum. Tthe kits can contain one or more vials in accordance with any of the foregoing, wherein each vial contains a single unit dose for administration to a subject. The kits can comprise lyophilates, disposed as above, that upon reconstitution provide compositions in accordance therewith. The kits can contain a lyophilate and a sterile diluent for reconstituting the lyophilate.

The present specification discloses a kit comprising in one or more containers an acetic buffer having a pH from about 4.0 to about 6.0 or an appropriate acetate salt to prepare such a buffer, proline at a concentration of about 2% to about 10%, and an antibody or antigen-binding fragment, wherein the kit does not further comprise both a polyol and a surfactant, and instructions regarding the use thereof.

Embodiments of the invention are not to be limited in scope by the specific embodiments described herein which are intended as illustrations of embodiments of the invention, and any compositions or methods which are functionally equivalent are within the scope of this invention. Indeed, various modifications of the invention in addition to those shown and described herein will become apparent to those skilled in the art from the foregoing description and accompanying drawings. Such modifications are intended to fall within the scope of the appended claims.

The following examples are intended merely to illustrate embodiments of the invention. The examples are according to the invention if they fall under the claims. All other examples are reference examples.

### Example 1. Formulation Stability

To determine the effects of different formulations on the stability of an antibody, several formulations as shown in Table 2 below were prepared. The formulations were stored in 5 cc blow back, Type 1 glass vials with rubber Daikyo fluoropolymer stoppers containing approximately 3.0 ml of formulated antibody solution for various times at different temperatures.

**Table 2: Formulations**

| Name | Buffer | pH | Excipient (w/v) | Concentration (mg/ml) |
|---|---|---|---|---|
| E51Su30 | 10mM L-glutamic acid | 5.1 | 8.35% sucrose | 30 |
| E51S30 | 10mM L-glutamic acid | 5.1 | 5% sorbitol | 30 |
| E51T30 | 10mM L-glutamic acid | 5.1 | 8.35% trehalose | 30 |
| E51G30 | 10mM L-glutamic acid | 5.1 | 2.5% glycerol | 30 |
| E51M130 | 10mM L-glutamic acid | 5.1 | 8.35% maltose | 30 |
| E51A30 | 10mM L-glutamic acid | 5.1 | 2.8% L-arginine HCl | 30 |
| E51M30 | 10mM L-glutamic acid | 5.1 | 5mM methionine + 2.45% glycerol | 30 |
| E51Gly30 | 10mM L-glutamic acid | 5.1 | 2.0% glycine | 30 |
| E51P30 | 10mM L-glutamic acid | 5.1 | 3.1% L-proline | 30 |
| E51L30 | 10mM L-glutamic acid | 5.1 | 2.5% L-lysine HCl | 30 |
| E51N30 | 10mM L-glutamic acid | 5.1 | 0.79% NaCl (135mM) | 30 |
| E51Mg30 | 10mM L-glutamic acid | 5.1 | 10mM MgCl₂, 2.22%glycerol | 30 |
| E51EDTA30 | 10mM L-glutamic acid | 5.1 | 2mM EDTA, 2.5% glycerol | 30 |
| A51G30 | 10mM acetic acid | 5.1 | 2.5% glycerol | 30 |
| A51A30 | 10mM acetic acid | 5.1 | 5.7% L-arginine | 30 |
| D51G30 | 10mM L-aspartic acid | 5.1 | 2.5% glycerol | 30 |
| D51A30 | 10mM L-aspartic acid | 5.1 | 5.7% L-arginine | 30 |

A formulation comprising an antibody of interest, for example, an antibody to NGF (an IgG₂) was prepared. Methods of preparing an antibody to NGF are known in the art and can be found, for example, in US Patent Application Publication 2005/0074821. The formulations comprised an aqueous solution of L-glutamic acid, L-aspartic acid or acetic acid buffer. The buffer had a concentration of 10 mM and a pH of 5.1, and the antibody was present at a concentration of 30 mg/ml. Additional components were added to each formulation as described above. E51P30 contained L-glutamic acid, proline and an antibody to nerve growth factor.

Different methods for determining stability of the antibody to nerve growth factor were used. The methods included size exclusion chromatography (SEC), cation exchange chromatography (CEX) and particle counting. Generally, the methods were performed as follows.

SEC was performed on an Agilent 1100 Capillary HPLC system equipped with a UV diode array detector, cooled autosampler, a normal flow cell and temperature controlled column compartment (Agilent, Palo Alto, CA, USA). The mobile phase included water with 100 mM sodium phosphate (Amgen Spec Number S2700R01), 330 mM NaCl (Amgen Spec Number S2706R02) at pH 6.6. Phenomenex Shodex KW-803 column (300 x 8 mm) was used for the SE analysis (Phenomenex, Torrance, CA, USA). Column compartment temperature was held at 25°C and the flow rate was 0.5 mL/min.

CEX was performed on an Agilent 1100 Capillary HPLC system equipped with a UV diode array detector, cooled autosampler, a normal flow cell and temperature controlled column compartment (Agilent, Palo Alto, CA, USA). The mobile phase included water with 10mM sodium phosphate (Amgen Spec Number S2700R01) pH 7.4 in solvent A and 10mM sodium phosphate (Amgen Spec Number S2700R01), 250 mM NaCl ((Amgen Spec Number S2706R02) pH 7.4 in solvent B. A weak-cation exchange column (Dionex ProPac WCX-10 column -- 4 x 250 mm, Dionex, Sunnyvale, CA, USA was used. Column compartment temperature was held at 25°C and the flow rate was 0.8 mL/min.

Subvisible particle analysis using a light obscuration technique was conducted with a HIAC Royco, liquid particle counting system, Model 9703 (Hach-Ultra, Grants Pass, OR, USA). The instrument was calibrated with a 15 um EZY-CAL standard (catalog No. 6015, Duke Scientific, Palo Alto, CA, USA). All formulations were degassed for 3 hours prior to the analysis. The instrument was cleaned between samples with deionized H₂O or formulation buffer. The number of particles was measured by performing four 0.5 ml draws of each antibody formulation using a 1 ml syringe.

Antibodies to NGF were prepared by methods known in the art (for example, *see* US Patent Application Publication 20050074821) and formulations comprising the antibodies were made. The NGF antibody used in the formulations was an IgG₂ antibody. The formulations were stored for various periods or were subjected to repeated freeze-thaws. Analysis of these formulations is presented in Figures 1A-1D, 2A-2F, and 3A-3C, all of which contain an antibody that binds to NGF.

Figure 1A-1D provides results of SEC analysis for the different formulations described above in Table 1 at 4°C, 25°C, 37°C and after repeated freezing (-30°C) and thawing at room temperature. In some experiments the solutions were stored for as long as 18 months and then analyzed. The formulations contained an antibody to NGF at a concentration of 30 mg/ml, 10 mM L-glutamic acid buffer, 10 mM L-apartic acid buffer or 10 mM acetic acid buffer at pH 5.1 and other components as indicated in the table.

SEC was used to provide information concerning stability of the antibody as measured by aggregation of the antibody during storage. The data is presented as a percentage of the main peak (monomer) - the greater the percentage of the main peak, the less aggregation that occurred. The proline-containing formulation EP1P30 usually provided better stability for stored antibody solutions than other formulations, however.

The proline-containing preparation, preparation E51P30, contained 10 mM L-glutamic acid buffer (pH 5.1), 3.1% proline and 30 mg/ml of antibody. Size exclusion chromatography can provide information relating to stability of a biopharmaceutical in a formulation in terms of aggregation. The greater the percentage of the main peak at each time in the figure, the less aggregation (including dimer and other high molecular weight aggregates) that has occurred.

Figure 1A illustrates results following storage at 4°C. Figure 1B illustrates results following storage at 25°C. Figure 1C illustrates results following storage at 37°C. Figure 1D illustrates results following the repeated freezing at -30°C and thawing at room temperature. Using SEC, the results demonstrate that generally the E51P30 formulation displays the least loss in percentage of the main peak over storage time, although some formulations may show comparable results for a specific time period. The lower loss in percentage of the main peak over storage time is evident following storage at 4°C for 12 or 18 months (Fig 1A), 25°C for 8 weeks, 13-weeks or 6-months at 25°C (Fig. 1B), and particularly for 6 months at 37°C (Fig. 1C). It should be noted, however, that the E51T30 and E51M30 formulations may also provide increased stability at certain temperatures.

Figure 1D shows that generally comparable results are obtained from the different formulations following repeated freeze-thaw cycles.

A characteristic relating to stability of stored antibodies and other polypeptides can be the occurrence of insoluble protein aggregates (referred to as particles hereafter). In this context, a proteinaceous particle refers to, for example, a fragment or aggregate of the insoluble polypeptide and can be visible and/or sub-visible. Particles can alternatively comprise matter that is foreign (e.g., shards of glass, lint, small pieces of rubber stopper) and not necessarily composed of the polypeptide. These foreign particles are not derived from antibodies and other polypeptides and no foreign particles were observed in the formulation described in these experiments. Soluble protein aggregates can be evaluated, for example, using methods such as SEC, whereas proteinaceous particles that are insoluble can be evaluated using such methods as liquid particle counting or turbidimetric techniques (empirical light scattering approach), for example.

Visible particles are generally classified as particles having sizes greater than 100 µm. Sub-visible particles, considered fine particles, are smaller in size. Using an LD-400 laser system with a HIAC instrument, particle sizes between 2 and 400 µm can be measured.

Figures 2A-2F provides data obtained from analysis of particle formation (>10 µm or >25 µm) following storage of the various formulations at 4°C, 25°C and 37°C. The data presented in Figures 2A-2D illustrates the measured assessment based on the number of particles per ml. In many instances, there are fewer particles/ml found in proline-containing formulations except at 18 months at 4°C. An good example of this is the number of 10 µm particles/ml following storage at >25°C for 13 weeks (*See* Figure 2C). Another example is the number of >10 µm particles/ml following storage at 37°C for 4 weeks (*See* Figure 2E).

Chemical modifications of polypeptides in the formulations were determined by cation exchange chromatography (CEX) as described above. This method separated isoforms based on protein surface charge differences using a linear salt gradient at pH 7.4 and a weak-cation exchange column (Dionex, WCX-10; Sunnyvale, CA).

Figures 3A-3B provides data obtained from analysis of particle formation (>10 µm or >25 µm) following 5 cycles of freeze-storage. Formulations were stored at -30°C. In several instances, formulations comprising proline were as good and sometimes better than other formulations in terms of lack of particle formation (particle size >10 µm).

Figures 4A-4C illustrates the results of cation exchange chromatography. Changes in the percentage of the main peak (Peak 0) after incubation at 4°C, 25°C and 37°C were analyzed. The decrease in the main peak is accompanied by increase in acidic peaks (data not shown). These changes are caused by chemical modifications (for example, deamidation) of the molecule. The data shows that the formulation containing L-proline (E51P30) is among the best in terms of maintaining the highest percentage of the main peak after incubation at all three temperatures.

### Example 2. Formulation Stability in Vials and Pre-Filled Syringes

Stability of various formulations during storage in either vials or pre-filled syringes was investigated. Stability of a formulation comprising an antibody was examined after 1 week, 2 weeks, 1 month and two months at 37°. The formulations were stored in vials or pre-filled syringes for various times at different temperatures. Table 3 lists several different formulations used in this study.

**Table 3.**

| Name | Buffer | pH | Excipient (w/v) | Concentration (mg/ml) |
|---|---|---|---|---|
| A52P_40 | 30mM acetic acid | 5.2 | 2.65% L-proline | 40 |
| A52PT006_40 | 30mM acetic acid | 5.2 | 2.65% L-proline, 0.006% polysorbate-20 | 40 |
| A52PT01_40 | 30mM acetic acid | 5.2 | 2.65% L-proline, 0.01% polysorbate-20 | 40 |
| A52SuT006_40 | 30mM acetic acid | 5.2 | 7.15% sucrose, 0.006% polysorbate-20 | 40 |
| A52GT006_40 | 30mM acetic acid | 5.2 | 2.14% glycerol, 0.006% polysorbate-20 | 40 |
| D52PT006_40 | 30mM L-aspartic acid | 5.2 | 2.65% L-proline, 0.006% polysorbate-20 | 40 |
| D52GT006_40 | 30mM L-aspartic acid | 5.2 | 2.14% glycerol, 0.006% polysorbate-20 | 40 |
| D52SuT006_40 | 30mM L-aspartic acid | 5.2 | 7.15% sucrose, 0.006% polysorbate-20 | 40 |
| E52PT006_40 | 30mM L-glutamic acid | 5.2 | 2.65% L-proline, 0.006% polysorbate-20 | 40 |
| E52GT006_40 | 30mM L-glutamic acid | 5.2 | 2.14% glycerol, 0.006% polysorbate-20 | 40 |
| SBPT006_40 | -- | 5.2 | 3.32% L-proline, 0.006% polysorbate 20 | 40 |

Another set of experiments was performed using a different set of formulations (Table 3). All formulations except one (SBPT006_40) contained at least one of 30 mM acetic acid buffer, 30 mM aspartic acid buffer, or 30 mM glutamic acid buffer, at pH 5.2 and an antibody to nerve-growth factor at a concentration of 40 mg/ml. SBPT006_40 contained 3.32% proline and 40 mg/ml antibody and had a pH of 5.2.

SBPT006_40 is a self-buffering solution and did not contain buffering reagent in the solution - no glutamic acid, aspartic acid or acetic acid buffer. Various embodiments are drawn to a formulation consisting of or consisting essentially of proline and an antibody, for example an antibody to nerve-growth factor. The proline and antibody can be in an aqueous solution. Additional information concerning self-buffering formulations can be found in PCT/US2006/022599.

Figures 5A-5H illustrates comparative data for storage in vials and pre-filled syringes stored at 37°C or 25°C. Figure 5A-5D provide results using antibody at 40 mg/ml while Figure 5E-5H provides results using antibody at 3 mg/ml. The main peak percent areas obtained for different formulations studied in glass vials and pre-filled syringes are presented. Formulations containing L-proline, irrespective of the buffering agent used, showed better stability in terms of the highest percent of main peak observed by SEC after incubating samples at 25°C or 37°C for two months. In Figures 5A-5D, the formulation SBPT006_40 (a self-buffering formulation containing proline with 40 mg/ml of antibody) consistently provided the best results whether in a vial or a pre-filled syringe.

Figures 5E-5F provides results at a lower antibody concentration (3mg/ml). At this concentration of antibody, some proline-containing formulation do not appear to maintain the stability to the same extent as non-proline formulations. It should be noted,, however, that several of the proline containing formulations provide stability equal to or better than non-proline containing formulations. While A52P_03 appears to provide the best stability relative to almost all the other formulations, visible particulation was observed in this formulation since it did not contain any polysorbate-20.

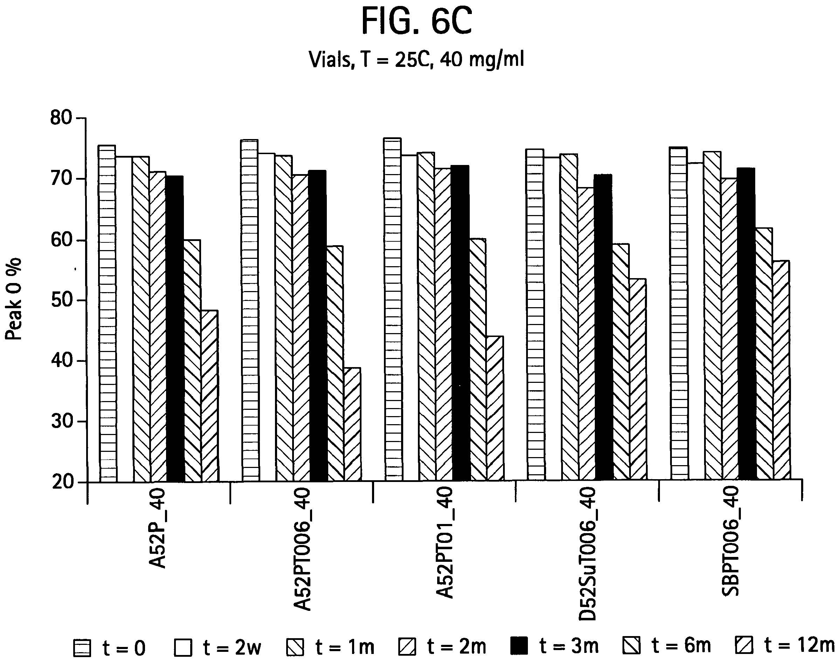

Figures 6A-6D shows results from cation exchange chromatography obtained for different formulations studied in glass vials and pre-filled syringes. At 37°C the decrease in percentage of Peak-0 appears similar most formulations of Figure 6A-6B. In the vials, however, A52P_40 shows the best results after 3 months, while in pre-filled syringes SBPT006_40 shows the best results at the same time period. Similarly, SBPT006_40 shows the best results after 12 months at 25°C in vials (Figure 6C)

Figures 6E-6H provides data from experiments in vials and pre-filled syringes using an antibody concentration of 3 mg/ml. The formulations are similar to those described in Table 3 except that a lower antibody concentration is used. As such, the formulation of A52P_03 is the same as A52P_40 except with 3 mg/ml of antibody rather than 40 mg/ml. In almost all instances A52P_03, a proline-containing formulation, provides greater stability at either two or three months storage in both vials and pre-filled syringes, however, this formulation can be prone to particulation due to the absence of polysorbate-20.

Table 4 provides a description of formulations used in Figures 7A-7B. The figures provide results from storage of formulations at -30°C using an antibody concentration of 40 mg/ml.

**Table 4**

| | | |
|---|---|---|
| A52P | 30 mM acetate, 2.6% proline | pH 5.2 |
| D52P | 30 mM L-aspartic acid, 2.6% proline | pH 5.2 |
| D52G | 30 mM L-aspartic acid, 2.1% glycerol | pH 5.2 |
| E52P | 30 mM L-glutamic acid, 2.6% L-proline | pH5.2 |
| E52G | 30 mM L-glutamic acid, 2.1% glycerol | pH 5.2 |

Formulations were either stored continuously (Figure 7A) or underwent five cycles of freezing and thawing (Figure 7B). In can be seen in Figure 7A, that at 12 months storage, the proline-containing solutions provided increased stability relative to the formulations without proline. This was also true following several freeze-thaws.

Figures 8A-8G and 9A-9B provide results obtained using an IgG₁ interleukin antibody. A pH 5.2 acetate buffer with antibody at 100 mg/ml was used in all formulations with the addition of components indicated in the Figure. All excipients used in these formulations are at a concentration of 270 mM except PEG-6000 which is at a concentration of 2% (w/v)

In all experiments in Figures 8A-8G, a proline-containing formulation contained either fewer aggregates (Figures 8A or 8C) or demonstrated increased percentage of the main peak (Figures 8B, 8D and 8E) reflecting increased stability of the antibody in the formulation. Figure 8E represents represents a zoom of Figure 8D for the period of 0-6 months.

Figures 9A-9D provide results in sodium acetate buffer at pH 5.2 comparing a proline-containing formulation with surfactant against a sorbitol-containing formulation with a surfactant. Sorbitol and proline are at 270 mM and both formulations contain 0.004% polysorbate 20. It can be seen from the figures that results of proline and surfactant are better than those with sorbitol and a surfactant at either 4°C or 29°C.

All of the above demonstrates that in most instances, a proline containing formulation produces either increased stability for long-term storage of antibody-containing solutions or is at least comparable to a non-proline containing formulation. As such, proline-containing solutions provide novel and new formulations for long-term storage of antibody-containing solutions.

Throughout this specification various publications, patents and patent applications have been referenced. The reference to such documents, however, should not be construed as an acknowledgment that such documents are prior art to the application. Further, merely because a document has been referenced, this does not necessarily indicate that the applicants are in complete agreement with the document's contents.

Although various embodiments of the invention have been described with reference to various embodiments, those skilled in the art will readily appreciate that the specific examples and studies detailed above are only illustrative.

## Claims

1. A formulation comprising an acetic acid buffer having a pH from about 4.0 to about 6.0, proline at a concentration of about 2% to about 10% (w/v), and an antibody or antigen-binding fragment, wherein the formulation does not further comprise both a polyol and a surfactant.

2. The formulation of claim 1, wherein the buffer is at a concentration of about 5 mM to about 50 mM.

3. The formulation of claim 1, wherein the buffer is at a concentration of about 10 mM, about 30 mM, or about 50 mM and has a pH of about 5.

4. The formulation of claim 1, wherein the antibody or antigen-binding fragment is at a concentration of about 50 mg/ml to about 100 mg/ml.

5. The formulation of claim 1, wherein the antibody or antigen-binding fragment is at a concentration of greater than 50 mg/ml.

6. The formulation of claim 1, wherein the antibody or antigen-binding fragment is at a concentration of greater than about 100 mg/ml.

7. A method of preparing a formulation comprising combining an acetic acid buffer having a pH from about 4.0 to about 6.0, proline and an effective amount of an antibody or antigen-binding fragment, wherein the formulation does not further comprise both a polyol and a surfactant.

8. A container containing a formulation comprising an aqueous solution having between about 3 to about 50 mM acetic acid with a pH from about 4.0 to about 6.0, proline from about 2% to about 10% (w/v) and an antibody or antigen-binding fragment, wherein the aqueous solution does not further comprise both a polyol and a surfactant.

9. The container of claim 8, wherein the container is a vial or a pre-filled syringe.

10. A formulation for use in treating a condition caused by increased expression of nerve growth factor or increased sensitivity to nerve growth factor in a patient, comprising an acetic acid buffer having a pH from about 4.0 to about 6.0, proline at a concentration of about 2% to about 10% (w/v), and an effective amount of an antibody or antigen-binding fragment to nerve-growth factor, wherein the formulation does not further comprise both a polyol and a surfactant.

## Patentansprüche

1. Formulierung, die einen Essigsäurepuffer mit einem pH-Wert von etwa 4,0 bis etwa 6,0, Prolin in einer Konzentration von etwa 2% bis etwa 10% (w/v), und einen Antikörper oder ein Antigen-bindendes Fragment umfasst, wobei die Formulierung weiter nicht sowohl ein Polyol als auch ein Tensid umfasst.

2. Formulierung nach Anspruch 1, wobei der Puffer eine Konzentration von etwa 5 mM bis etwa 50 mM aufweist.

3. Formulierung nach Anspruch 1, wobei der Puffer eine Konzentration von etwa 10 mM, etwa 30 mM oder etwa 50 mM aufweist, und einen pH-Wert von etwa 5 hat.

4. Formulierung nach Anspruch 1, wobei der Antikörper oder das Antigen-bindende Fragment in einer Konzentration von etwa 50 mg/ml bis etwa 100 mg/ml vorliegt.

5. Formulierung nach Anspruch 1, wobei der Antikörper oder das Antigen-bindende Fragment in einer Konzentration von mehr als 50 mg/ml vorliegt.

6. Formulierung nach Anspruch 1, wobei der Antikörper oder das Antigen-bindende Fragment in einer Konzentration von mehr als etwa 100 mg/ml vorliegt.

7. Verfahren zur Herstellung einer Formulierung, umfassend das Kombinieren eines Essigsäurepuffers mit einem pH-Wert von etwa 4,0 bis etwa 6,0, Prolin und einer wirksamen Menge eines Antikörpers oder eines Antigen-bindenden Fragments, wobei die Formulierung weiter nicht sowohl ein Polyol als auch ein Tensid umfasst.

8. Behälter, umfassend eine Formulierung, die eine wässrige Lösung mit zwischen etwa 3 bis etwa 50 mM Essigsäure mit einem pH-Wert von etwa 4,0 bis etwa 6,0, Prolin von etwa 2% bis etwa 10% (w/v) und einen Antikörper oder ein Antigen-bindenden Fragments enthält, wobei die wässrige Lösung weiter nicht sowohl ein Polyol als auch ein Tensid enthält.

9. Behälter nach Anspruch 8, wobei der Behälter eine Ampulle oder eine vorgefüllte Spritze ist.

10. Formulierung zur Verwendung bei der Behandlung einer Erkrankung, die durch eine erhöhte Expression von Nervenwachstumsfaktor oder eine erhöhte Empfindlichkeit gegenüber dem Nervenwachstumsfaktor bei einem Patienten verursacht ist, umfassend einen Essigsäurepuffer mit einem pH-Wert von etwa 4,0 bis etwa 6,0, Prolin in einer Konzentration von etwa 2% bis etwa 10% (w/v), und eine wirksame Menge eines Antikörpers oder eines Antigen-bindenden Fragments gegen den Nervenwachstumsfaktor, wobei die Formulierung weiter nicht sowohl ein Polyol als auch ein Tensid umfasst.

## Revendications

1. Formulation comprenant un tampon acide acétique ayant un pH d'environ 4,0 à environ 6,0, de la proline sous une concentration d'environ 2 % à environ 10 % (pds/vol), et un anticorps ou un fragment de liaison à l'antigène, la formulation ne comprenant à la fois ni polyol ni tensioactif.

2. Formulation selon la revendication 1, le tampon se trouvant sous une concentration d'environ 5 mM à environ 50 mM.

3. Formulation selon la revendication 1, le tampon se trouvant sous une concentration d'environ 10 mM, d'environ 30 mM, ou d'environ 50 mM et présentant un pH d'environ 5.

4. Formulation selon la revendication 1, l'anticorps ou le fragment de liaison à l'antigène se trouvant sous une concentration d'environ 50 mg/ml à environ 100 mg/ml.

5. Formulation selon la revendication 1, l'anticorps ou le fragment de liaison à l'antigène se trouvant sous une concentration supérieure à 50 mg/ml.

6. Formulation selon la revendication 1, l'anticorps ou le fragment de liaison à l'antigène se trouvant sous une concentration supérieure à environ 100 mg/ml.

7. Procédé de préparation d'une formulation comprenant la combinaison d'un tampon acide acétique ayant un pH d'environ 4,0 à environ 6,0, de la proline et une quantité efficace d'un anticorps ou d'un fragment de liaison à l'antigène, la formulation ne comprenant à la fois ni polyol ni tensioactif.

8. Conteneur contenant une formulation comprenant une solution aqueuse ayant de l'acide acétique d'environ 3 à environ 50 mM avec un pH d'environ 4,0 à environ 6,0, de la proline d'environ 2 % à environ 10 % (pds/vol) et un anticorps ou un fragment de liaison à l'antigène, la solution aqueuse ne comprenant à la fois ni polyol ni tensioactif.

9. Conteneur selon la revendication 8, le conteneur étant un flacon ou une seringue pré-rempli-e.

10. Formulation pour l'utilisation dans le traitement d'un état provoqué par l'expression accrue d'un facteur de croissance des nerfs ou une sensibilité accrue au facteur de croissance des nerfs chez un patient, comprenant un tampon acide acétique ayant un pH d'environ 4,0 à environ 6,0, de la proline sous une concentration d'environ 2 % à environ 10 % (pds/vol), et une quantité efficace d'un anticorps ou d'un fragment de liaison à l'antigène au facteur de croissance des nerfs, la formulation ne comprenant à la fois ni polyol ni tensioactif.
